# EUROPEAN PATENT APPLICATION

(11) **EP 4 194 010 A1**
(43) Date of publication of application: **14.06.2023**
(21) Application number: 21852799.2
(22) Date of filing: 04.08.2021
(51) Int. Cl.: A61K 47/54, A61K 45/06, A61P 35/00, A61P 37/00, A61K 47/68, A61K 39/39, A61K 39/00

(54) **CONJUGATE OF FUNCTIONAL DRUG AND TOLL-LIKE RECEPTOR 7 OR 8 AGONIST OF WHICH ACTIVE SITE IS TEMPORARILY INACTIVATED AND USE THEREOF**

(30) Priority: 04.08.2020 KR 20200097550; 04.08.2020 KR 20200097553; 03.08.2021 KR 20210102226
(71) Applicant: PROGENEER INC., Guro-gu Seoul 08380 (KR)
(72) Inventor: LIM, Yong Taik, Seongnam-si, Gyeonggi-do 13597 (KR); LEE, Sang Nam, Suwon-si Gyeonggi-do 16357 (KR); JIN, Seung Mo, Seoul 06709 (KR)
(74) Representative: Dehns
(86) International application number: PCT/KR2021/010298
(87) International publication number: WO 2022/031057

(57) **Abstract**

The present invention relates to: a toll-like receptor 7 or 8 agonist-drug complex material in which a functional drug and a toll-like receptor 7 or 8 agonist are bonded by a chemical bond containing a cleavable site; and using the properties of the temporarily inactivated state, the side effects of the toll-like receptor 7 or 8 agonist can be reduced. In particular, the complex material is designed such that the immunostimulatory function of the toll-like receptor 7 or 8 agonist is inhibited due to the chemical bond, while the immunostimulatory function is recovered at an injection site or in a tumor microenvironment or target immune cells, thus exhibiting kinetic properties in which immunostimulatory efficacy can be controlled over time, and minimizing the induction of nonspecific immune responses. In addition, since various functions can be combined with a drug, the inactivated toll-like receptor 7 or 8 agonist-functional drug complex can be widely used in pharmaceutical compositions for various applications, which are capable of controlling cancer cell death, the activation of therapeutic immune cells, and immunosuppressive cells and environments, at an injection site or in a tumor microenvironment.

## Description

### [Technical Field]

The present invention relates to a complex in which a functional drug binds to the active site of a toll-like receptor 7 or 8 agonist, and more particularly, to a complex in which the function of a toll-like receptor 7 or 8 agonist is temporarily inhibited by connecting a functional drug to the active site of the toll-like receptor 7 or 8 agonist and is then kinetically recovered a predetermined time later, and a use thereof.

### [Background Art]

Innate immune responses in the body play a very important role in the prevention or treatment of various infectious diseases, cancer, metabolic diseases, or rare diseases. However, patients with specific diseases often have reduced normal immune response function. Accordingly, recently, as a method applicable to the prevention, improvement, and treatment of various diseases, various functional drugs that strengthen weakened immune responses in the body or reactivate immune responses inhibited or stopped by a specific signal have been developed. Generally, an immune response is a series of reactions caused by activated immune cells against foreign and endogenous materials, i.e., antigens, and when microorganisms, including bacteria and viruses, or foreign substances in the living organism are introduced *in vivo,* immune cells recognize them to activate and secrete a factor such as a cytokine, thereby causing an inflammatory response. Recently, studies on mechanisms at the innate immune response stage that non-specifically act at the beginning of infection are actively progressing, and among them, a toll-like receptor is a receptor capable of recognizing a pathogen at the early stage of inflammation, and it is known to induce an immune response by recognizing the plasma membrane component or nucleic acid component of a pathogen, and using this, research on various toll-like receptor ligands for activating immune cells is actively progressing.

Among various toll-like receptor ligands, a toll-like receptor 7 or 8 agonist-based material is used as an adjuvant inducing a cellular immune response, and an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist are known (US Patent Application Publication No. 2012-0294885). Such a toll-like receptor 7 or 8 agonist is known to effectively induce not only humoral immunity but also cellular immunity as a toll-like receptor 7 or 8 agonist in an endosome. In addition, recently, it has been additionally reported that since a toll-like receptor 7 or 8 agonist performs an immune suppressive action in an injection site or tumor microenvironment beyond the function of an adjuvant, the function of immune suppressor cells helping cancer growth and metastasis, such as MDSCs, Tregs, or M2 macrophages, is inhibited, resulting in improvement of an immune anti-cancer effect. However, such multifunctional toll-like receptor agonists are difficult to be dispersed in an aqueous solution due to their molecular structure. In addition, since they are dissolved only in specific organic solvents such as DMSO and methanol, and not dissolved in generally used organic solvents, there are limitations in manufacturing immune-activating drugs in various formulations. Therefore, a cream-type formulation (e.g., Aldara cream) in which various surfactants were mixed are commercialized. In some studies, to overcome the above problems, such an agonist is prepared in the form of a salt and thus can be dissolved in an aqueous solution. However, the toll-like receptor agonist prepared in the form of a salt is absorbed into the blood vessel in the body to induce an immune response in the blood vessel (systemic immune response), thereby causing many side effects (e.g., cytokine storm, various non-specific hypersensitive immune responses, etc.), so it is not easy to use. In addition, due to such side effects, to be actually used in treatment, it has to be treated at a smaller concentration than the effective dose, so it also becomes a factor in decreasing efficacy. To overcome such problems, some pharmaceutical companies are trying to prevent directly absorption into blood vessels by introducing a lipophilic lipid, or by direct chemical bonding to a polymer chain having a large size. However, since the toll-like receptor agonist prepared by such a method has its active site still exposed to the outside, it induces non-specific immune response in the body and still has the possibility of inducing toxicity.

Therefore, if a toll-like receptor 7 or 8 agonist can be prepared to exhibit a kinetic characteristic capable of controlling immune activation efficacy depending on a time point by designing it to recover an immune activation function in an injection site, tumor microenvironment or target immune cells while inhibiting the immune activation function of the toll-like receptor 7 or 8 agonist due to a chemical bond of the active site at first, nonspecific toxicity and induction of an immune response by the toll-like receptor 7 or 8 agonist may be minimized. In addition, by combining with various functional drugs (antibodies, small molecule anticancer agents, antigens, cytokines, peptides, amino acids, low molecular compounds, lipids, oligonucleotides, targeting ligands, etc.), using the synergistic effect of the functional drugs and the toll-like receptor 7 or 8 agonist material, such a toll-like receptor 7 or 8 agonist material is expected to be widely used in pharmaceutical compositions for various purposes, which can specifically control cancer cell death, activation of therapeutic immune cells, immune suppressor cells and an environment at an injection site or in a tumor microenvironment.

### [Disclosure]

### [Technical Problem]

To solve the problems in the related art, the present invention is directed to providing a functional drug-toll-like receptor 7 or 8 agonist complex in which a toll-like receptor 7 or 8 agonist and a functional drug are connected, which is designed to temporarily inhibit the immune activation function of the toll-like receptor 7 or 8 agonist, and recover the immune activity specifically at an injection site, or in a tumor microenvironment or target cells, and a use thereof.

However, technical problems to be solved in the present invention are not limited to the above-described problems, and other problems which are not described herein will be fully understood by those of ordinary skill in the art from the following descriptions.

### [Technical Solution]

The present invention provides a toll-like receptor 7 or 8 agonist-functional drug complex, which is characterized by connecting a functional drug to the active site of a toll-like receptor 7 or 8 agonist. More particularly, the complex is a complex that has a toll-like receptor 7 or 8 agonist which is temporarily inactive because one end of a cleavable linker is chemically bonded to the active site of the toll-like receptor 7 or 8 agonist, that is, the active site exhibiting the biological activity of the toll-like receptor 7 or 8 agonist, and a functional drug which is chemically bonded to the other end of the linker.

In one embodiment of the present invention, in the complex, the functional drug primarily acts, and as the active site is kinetically recovered by cleaving the linker at an injection site, or in a tumor microenvironment or target cells, the inactivated toll-like receptor 7 or 8 agonist secondarily acts.

In another embodiment of the present invention, the chemical bond of the cleavable linker is cleaved by any one or more factors selected from the group consisting of an enzyme, pH, redox potential, a temperature, ultrasonic wave, magnetic force, and a light source.

In still another embodiment of the present invention, the cleavable linker is preferably any one or more selected from the group consisting of a benzyl elimination-based linker, a trialkyl lock-based linker, a bicine-based linker, an acid labile linker, a lysosomally cleavable peptide and a cathepsin B-cleavable peptide, and more preferably, a linker having any one or more bonds selected from the group consisting of carbamate, disulfide, hydrazine, ester, peptide, azide, and β-glucuronide bonds, and a combination thereof. However, the cleavable linker is not limited as long as it can chemically bind to the toll-like receptor 7 or 8 agonist and the functional drug to be connected with each other in a cleavable form, and has any form capable of cleaving the binding by the endogenous factors (enzyme, redox potential, GSH, pH, etc.) and/or exogenous factors (redox, pH, temperature, photo/light, magnetic, ultrasound, electrical responsive, etc.) of the body.

In yet another embodiment of the present invention, the cleavable linker further includes an alkyl derivative such as ethylene oxide or ethylene glycol at one or both ends, thereby increasing the solubility and flexibility of a complex in an aqueous solution.

In yet another embodiment of the present invention, the toll-like receptor 7 or 8 agonist may be any one or more selected from the group consisting of an imidazoquinoline-based agonist, a 8-hydroxyadenine-based agonist, a pteridone-based agonist, a 2-aminopyrimidine-based agonist, a benzoazepine-based agonist, and a 7-thia-8-oxoguanosine-based agonist, and it is not limited as long as it is any toll-like receptor 7 or 8 agonist which is inactivated by connecting a cleavable linker to an active site.

In yet another embodiment of the present invention, the functional drug may be any one or more selected from the group consisting of an antibody, an antibody fragment, a single-stranded antibody, an anticancer agent, an antigen, a cytokine, a protein, a peptide, an amino acid, an oligonucleotide, an enzyme, a lipid, a low-molecular compound, a glycoprotein, and a targeting ligand, and more preferably, any one or more selected from the group consisting of an antibody, a protein-based drug, a sensitizer, and an anticancer agent. However, it is not limited as long as it is a drug that can be connected with a toll-like receptor 7 or 8 agonist using a cleavable linker.

In addition, the present invention provides a composition for controlling immune system, which includes the complex as an active ingredient.

In one embodiment of the present invention, the composition for controlling immune system activates any one or more immune cells selected from the group consisting of antigen-presenting cells, B cells, natural killer (NK) cells, and T cells.

In another embodiment of the present invention, the composition for controlling immune system regulates the function of any one or more immune cells selected from the group consisting of regulatory T (Treg) cells, myeloid derived suppressor cells (MDSCs), and M2 macrophages.

In still another embodiment of the present invention, the composition for controlling immune system may induce the activation of immune cells at an injection site or in a tumor microenvironment or control the function of immune suppressor cells, and preferably induces immune activation of antigen-presenting cells (dendritic cells, macrophages, etc.), NK cells, or T cells, or regulates the immune system in the body by regulating the function of immune cells having an immunosuppressive action (Treg cells, MDSCs, or M2 macrophages).

In yet another embodiment of the present invention, the composition for controlling immune system may further include any one or more antibodies selected from the group consisting of anti-PD-1, anti-PD-L1, anti-CTLA-4, anti-KIR, anti-LAG3, anti-CD 137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT, which target immune checkpoint factors.

In addition, the present invention provides a pharmaceutical composition for preventing or treating cancer, which includes the complex as an active ingredient.

In one embodiment of the present invention, the pharmaceutical composition may further include materials generally used in treatment of cancer, such as a chemotherapeutic agent or an immune checkpoint inhibitor. The pharmaceutical composition include the complex of the present invention, and thus effectively activate the immune system in the body, thereby improving the efficacy of conventional chemotherapeutic agents or immune checkpoint inhibitors.

In another embodiment of the present invention, the pharmaceutical composition inhibits cancer growth, metastasis, recurrence or resistance to chemotherapy.

In still another embodiment of the present invention, the cancer may be, but is not limited to, breast cancer, colorectal cancer, rectal cancer, lung cancer, colon cancer, thyroid cancer, oral cancer, pharynx cancer, larynx cancer, cervical cancer, brain cancer, ovarian cancer, bladder cancer, kidney cancer, liver cancer, pancreatic cancer, prostate cancer, skin cancer, tongue cancer, uterine cancer, stomach cancer, bone cancer, or blood cancer.

In addition, the present invention provides a method of preventing or treating cancer, which includes administering a composition including the complex as an active ingredient into a subject.

In addition, the present invention provides a use of a composition including the complex as an active ingredient for preventing or treating cancer.

In addition, the present invention provides a use of the complex for the manufacture of medicament for preventing or treating cancer.

In addition, the present invention provides a method of preparing a functional drug-toll-like receptor 7 or 8 agonist complex, which includes a) chemically binding a cleavable linker to the active site of a toll-like receptor 7 or 8 agonist, and b) binding a functional drug to the linker.

### [Advantageous Effects]

As a functional drug acts primarily, and a toll-like receptor 7 or 8 agonist capable of inducing a synergistic effect therewith acts secondarily over certain period of time, a complex of a functional drug and a toll-like receptor 7 or 8 agonist whose activity is temporarily inhibited according to the present invention can minimize a nonspecific immune response and toxicity and maximize a synergistic effect. In addition, after delivery into an injection site, a tumor microenvironment or immune cells, the functional drug and the toll-like receptor 7 or 8 agonist are gradually separated, the active site of the toll-like receptor 7 or 8 agonist is kinetically modulated, and continuously reacts with the toll-like receptor for a long time as well as minimizing the toxicity of the toll-like receptor 7 or 8 agonist, so the persistence of the immune activation of immune cells may increase and thus the therapeutic effect may remarkably increase compared to when the toll-like receptor agonist is used alone. In addition, in the toll-like receptor 7 or 8 agonist that is chemically bound with a functional drug according to the present invention (functional drug-toll-like receptor 7 or 8 agonist complex), when an antibody is applied as the functional drug, side effects and cytotoxicity of the conventional toll-like receptor 7 or 8 agonist may be remarkably reduced by increasing the target efficiency to specific cells or tissue using the target specificity of the antibody. In addition, the death of cancer cells specifically targeted by an antibody and a cancer antigen made thereby may be combined with the adjuvant function of the toll-like receptor 7 or 8 agonist, thereby inducing a so-called in-situ vaccination effect. Moreover, since the functional drug-toll-like receptor 7 or 8 agonist complex may induce immune activation of antigen-presenting cells (dendritic cells, macrophages, or the like), NK cells, or T cells, and may regulate the function of immune cells having an immunosuppressive action (Treg cells, MDSCs, M2 macrophages, or the like) at an injection site, or in a tumor microenvironment, the complex alone may exhibit an anticancer effect, or when the complex is prepared using an immune checkpoint inhibitor or a chemotherapeutic agent as a functional drug, an anticancer effect may be remarkably increased due to a synergistic effect. Therefore, as the complex of the present invention is effectively applied to all functional drugs that can be co-administered with the toll-like receptor 7 or 8 agonist, side reactions can be reduced, and therapeutic effects can be enhanced, so that it is expected that the complex can be widely used in various fields.

### [Description of Drawings]

FIG. 1 shows representative linkers for connecting a toll-like receptor 7 or 8 agonist with a functional drug according to one embodiment of the present invention.
FIG. 2 shows representative anticancer agents having an amine group, an example of a complex of an amine group-containing anticancer agent and a toll-like receptor 7 or 8 agonist, having a bond cleaved by a reducing agent, and schematically shows mechanisms cleaved by a reducing agent according to one embodiment of the present invention.
FIG. 3 shows an example of a complex of an amine group-containing anticancer agent and a toll-like receptor 7 or 8 agonist, having a bond cleaved under an acidic condition, and schematically shows cleavage mechanisms according to one embodiment of the present invention.
FIG. 4 shows representative anticancer agents having a hydroxyl group and an example of a complex of an anticancer agent and a toll-like receptor 7 or 8 agonist, having a bond cleaved by β-glucuronidase, and schematically shows cleavage mechanisms according to one embodiment of the present invention.
FIG. 5 shows an example of a complex of a hydroxyl group-containing anticancer agent and a toll-like receptor 7 or 8 agonist, having a bond cleaved by a protease, and schematically shows cleavage mechanisms according to one embodiment of the present invention.
FIG. 6 shows representative linkers and linkage mechanisms for synthesizing a complex of a protein-based drug and a toll-like receptor 7 or 8 agonist according to one embodiment of the present invention.
FIG. 7 shows an example of a complex of a protein-based drug and a toll-like receptor 7 or 8 agonist, having a bond cleaved by a reducing agent, and schematically shows cleavage mechanisms according to one embodiment of the present invention.
FIG. 8 shows an example of a complex of a protein-based drug and a toll-like receptor 7 or 8 agonist, having a bond cleaved by the concentration of hydrogen ions in cells, and schematically shows cleavage mechanisms according to one embodiment of the present invention.
FIG. 9 shows an example of a complex of a protein-based drug and a toll-like receptor 7 or 8 agonist, having a bond cleaved by β-glucuronidase, and schematically shows cleavage mechanisms according to one embodiment of the present invention.
FIG. 10 shows an example of a complex of a protein-based drug and a toll-like receptor 7 or 8 agonist, having a bond cleaved by a reducing agent, and schematically shows cleavage mechanisms according to one embodiment of the present invention.
FIG. 11 shows an example of a complex of a protein-based drug and a toll-like receptor 7 or 8 agonist, having a bond cleaved by the concentration of hydrogen ions in cells, and schematically shows cleavage mechanisms according to one embodiment of the present invention.
FIG. 12 shows an example of a complex of a protein-based drug and a toll-like receptor 7 or 8 agonist, having a bond cleaved by β-glucuronidase, and schematically shows cleavage mechanisms according to one embodiment of the present invention.
FIG. 13 shows representative types of sensitizers, an example of a complex of a sensitizer and a toll-like receptor 7 or 8 agonist, having a bond cleaved by a reducing agent, and schematically shows cleavage mechanisms according to one embodiment of the present invention.
FIG. 14 shows an example of a complex of a carboxyl group-containing sensitizer and a toll-like receptor 7 or 8 agonist, having a bond cleaved under an acidic condition, and schematically shows cleavage mechanisms according to one embodiment of the present invention.
FIG. 15 shows an example of a complex of a carboxyl group-containing sensitizer and a toll-like receptor 7 or 8 agonist, having a bond cleaved by β-glucuronidase, and schematically shows cleavage mechanisms according to one embodiment of the present invention.
FIGS. 16 show a result of confirming the anticancer effect of an AAC-001 complex by a cancer size according to one embodiment of the present invention.
FIG. 17 shows a result of confirming the anticancer effect of an AAC-001 complex by a survival rate according to one embodiment of the present invention.
FIG. 18 shows a result of confirming the effect of an AAC-001 complex on the secretion of an inflammatory cytokine in blood according to one embodiment of the present invention.
FIG. 19 schematically shows the mechanism of an AAC-002 complex according to one embodiment of the present invention. More particularly, it is a schematic diagram illustrating an action mechanism of an antibody-toll-like receptor 7 or 8 agonist complex in cells, and shows a process of changing an inactivated toll-like receptor 7 or 8 agonist into an activated state by separating the antibody from the toll-like receptor 7 or 8 agonist by an intracellular environment.
FIG. 20 shows a result of confirming the effect of an AAC-002 complex on the secretion of an inflammatory cytokine in blood according to one embodiment of the present invention.
FIG. 21 shows a result of confirming that a protein antigen-resiquimod complex having a bond cleaved by cathepsin-B forms nanoparticles having a size of approximately 99.7 nm in an aqueous solution.

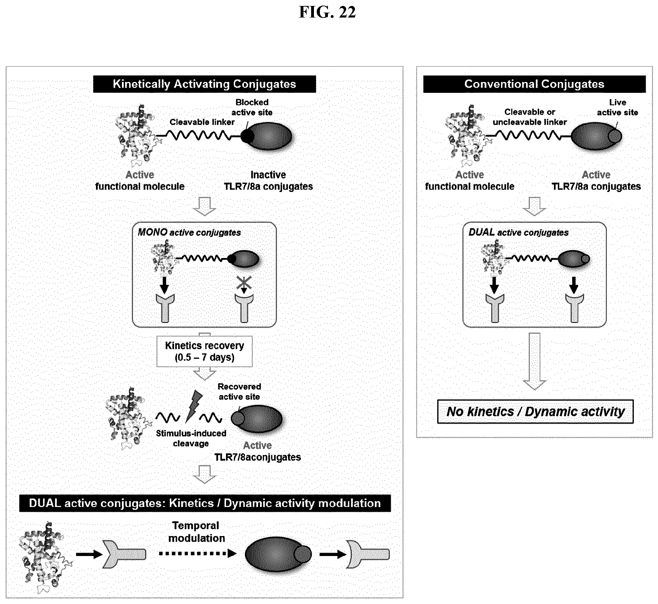

FIG. 22 is a schematic diagram showing the structure and function of a functional drug-toll-like receptor 7 or 8 agonist complex, in which the functional drug binds to the active site of the toll-like receptor 7 or 8 agonist by a cleavable linker, showing a difference between kinetically activating complexs, which are temporarily inactivated, and conventional complexs, in which a functional drug binds to another site, not the active site. In the kinetically activating complexs, the efficacy of a functional drug is effective at the beginning, and after the reaction of the cleavable linker, the efficacy of the inactivated toll-like receptor 7 or 8 agonist is kinetically regenerated secondarily in response to a stimulus, and such a kinetic action may not be realized with conventional complexs maintaining a dual active state of the functional drug and a toll-like receptor agonist from the beginning.

### [Best Modes of the Invention]

As a result of intensively conducting research to prepare a complex of a functional drug and a toll-like receptor 7 or 8 agonist, which reduces side effects as well as enabling co-administration with a single administration by connecting various functional drugs by inhibiting a nonspecific early immune response of the toll-like receptor 7 or 8 agonist, the present inventors invented a complex in which a functional drug binds to the active site of a toll-like receptor 7 or 8 agonist via a cleavable linker, so the functional drug primarily acts and then the toll-like receptor agonist whose immune activation action is temporarily inhibited acts secondarily due to the recovery of the immune activation function specifically at an injection site, or in a tumor microenvironment or target cells (FIG. 22). The inhibition may mean that the function of the toll-like receptor 7 or 8 agonist is delayed (retarded). The functional drug-toll-like receptor 7 or 8 agonist complex of the present invention differs from conventional complexs in that the activity of the functional drug and the toll-like receptor 7 or 8 agonist can be kinetically or dynamically modulated.

The "toll-like receptor agonist" used herein is a ligand directly or indirectly acting on a toll-like receptor, which is a membrane protein involved in innate immunity, and refers to a component that can cause a signaling response using a signaling pathway through the generation of an endogenous or exogenous ligand. The toll-like receptor agonist used herein may be a natural toll-like receptor agonist or a synthetic toll-like receptor agonist. The toll-like receptor agonist used herein may be a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, or a toll-like receptor 9 agonist. The toll-like receptor 7 or 8 agonist refers to a ligand that can induce a signaling response by means of TLR-7 or 8, and may be, but is not limited to, an imidazoquinoline-based agonist, a 8-hydroxyadenine-based agonist, a pteridone-based agonist, a 2-aminopyrimidine-based agonist, a benzoazepine-based agonist, or a 7-thia-8-oxoguanosine-based agonist, and the imidazoquinoline-based compound includes compound types disclosed in WO 2018 196823, WO 2011 049677, WO 2011 027022, WO 2017 102652, and WO 2019 040491, or pharmaceutically acceptable salts thereof, but the present invention is not limited thereto. In addition, the 8-hydroxyadenine-based compounds include compound types disclosed in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, and WO 2018 041763, or pharmaceutically acceptable salts thereof, but the present invention is not limited thereto. The pteridone-based compounds include compound types disclosed in US 2010 0143301, WO 2016 007765, WO 2016 044182, WO 2017 035230, WO 2017 219931, WO 2011 057148, and CN 1087 94486, or pharmaceutically acceptable salts thereof, but the present invention is not limited thereto. The 2-aminopyrimidine-based compounds include compound types disclosed in WO 2010 133885, WO 2012066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319 and WO 2013 117615, or pharmaceutically acceptable salts thereof, but the present invention is not limited thereto. The benzoazepine-based compounds include compound types disclosed in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112 and WO 2017 197624, or pharmaceutically acceptable salts thereof, but the present invention is not limited thereto. The 7-thia-8-oxoguanosine-based compounds include compound types disclosed in WO 2016 180691, WO 2016 055553, WO 2016 180743 and WO 2016 091698, or pharmaceutically acceptable salts thereof, but the present invention is not limited thereto. Other than the above, the toll-like receptor 7 or 8 compounds may also include toll-like receptor 7 or 8 compounds disclosed in PCT/US2009/035563, PCT/US2015/028264, PCT/US2016/020499, WO 2015 023598 and PCT/US 2015/039776, or pharmaceutically salt thereof. Alternatively, the toll-like receptor 7 or 8 agonists may include, but are not limited to, imiquimod, resiquimod, dactolisib, gardiquimod, sumanirole, motolimod, vesatolimod, loxoribine, SM360320, CL264, 3M-003, IMDQ, and Compound 54, and include all toll-like receptor 7 or 8 agonists which can be readily surmised by those of ordinary skill in the art. The toll-like receptor 7 or 8 agonists may be replaced with one or more immune-activating materials selected from the group consisting of a toll-like receptor agonist, saponin, an anti-viral peptide, an inflammasome inducer, a NOD ligand, a cytosolic DNA sensor (CDS) ligand, a stimulator of interferon genes (STING) ligand, an emulsion, alum, and a combination thereof, and the same concept may also be applied to a toll-like receptor 3 agonist or toll-like receptor 9 agonist having a receptor in an endosome after delivery into cells to synthesize a complex with an antibody, but the present invention is not limited thereto.

The "cleavable linker" used herein includes a cleavable bond, and is the generic term for linkers in which cleavage can occur due to conditions such as low pH, enzymes or glutathione in the body such as a tumor microenvironment or the physiological environments of endosomes and lysosomes in cells; or external stimuli, that is, specific stimuli such as a temperature, redox potential, ultrasound, magnetic field, and near-infrared light. The cleavable linker preferably means a linker including a carbamate, disulfide, hydrazine, ester, peptide, azide or β-Glucuronide bond, but there is no limitation as long as it has a cleavable form. In addition, in the complex of the present invention, the functional drug and the toll-like receptor 7 or 8 agonist may be separated by various enzymes present at an injection site, or in a tumor microenvironment or cells, such as acid phosphatase, acid pyrophosphatase, phosphodiesterase, phosphoprotein phosphatase, phosphatidic acid phosphatase, aryl sulfatase, proteases, cathepsins, collagenase, arylamidase, peptidase, acid ribonuclease, acid deoxyribonuclease, lipases, triglyceride lipase, phospholipase, esterases, carboxylesterase, glucocerebrosidase, galactocerebrosidase, sphingomyelinase, glycosidases, alpha-glucosidase, beta-glucosidase, beta-galactosidase, alpha-mannosidase, alpha-ucosidase, beta-xylosidase, alpha-N-acetylhexosaminidase, beta-N-acetylhexosaminidase, sialidase, lysozymes, hyaluronidase, beta-glucuronidase, and gamma-interferon-inducible lysosomal thiol reductase.

The "functional drug" used herein is the generic term for all drugs that have biological functions and thus can be used in diagnosis, prevention, and treatment of a disease, and is preferably an antibody, an antibody fragment, a single-stranded antibody, an anticancer agent, an antigen, a cytokine, a protein, a peptide, an amino acid, an oligonucleotide, an enzyme, a lipid, a low-molecular compound, a glycoprotein, or a targeting ligand, and more preferably, an anticancer agent, an antibody, a protein-based drug, or a sensitizer, but the present invention is not limited thereto. For example, the functional drug may be a DNA topoisomerase inhibitor, a microtubule inhibiting drug, a DNA damaging agent, an antimetabolite, a nucleoside analog, an antisense oligonucleotide, a locked nucleic acid (LNA), short interfering RNA (siRNA), microRNA (miRNA), an aptamer, a peptide nucleic acid (PNA), phosphorodiamidate morpholino oligonucleotide (PMO), antisense Bcl-2 oligonucleotide, antisense HIF-1α oligonucleotide, antisense Survivin oligonucleotide, a cell adhesion peptide, a cell penetrating peptide, a receptor ligand, a targeting carbohydrate molecule, lectin, an RGD peptide, selectin, TAT, penetratin, (Arg)9, or folic acid. The functional drug may also be a microtubulin structure synthesis inhibitor, a meiosis inhibitor, an RNA polymerase inhibitor, a topoisomerase inhibitor, a DNA intercalator, a DNA alkylator, a liposome inhibitor, a protein toxin, or a radioactive isotope, which is used in treatment of cancer. In addition, the functional drug may be maytansinoid, auristatin, dolastatin, tubulysin, calicheamicin, pyrrolobenzodiazepine, doxorubicin, duocamycin, carboplatin(paraplatin), cisplatin, cyclophosphamide, ifosfamide, nidran, nitrogen mustard (mechlorethamine HCL), bleomycin, mitomycin C, cytarabine, flurouracil, gemcitabine, trimetrexate, methotrexate, etoposide, vinblastine, vinorelbine, alimta, altretamine, procarbazine, taxol, taxotere, topotecan, irinotecan, trichothecene, CC1965, alpha-amanitin, an enediyne antibiotic, or a vegetable toxin. In addition, when the functional drug is a compound, a stereoisomer or derivative thereof may also be included. In addition, the auristatin may be monomethyl auristatin E or monomethyl auristatin F, and it is not limited as long as it is a material that is used in treatment of cancer. In addition, a sensitizer may be protoporphrin, hematoporphrin monomethryl ether, pheophorbide A, or photofrin, and it is not limited as long as it is a chemical sensitizer or optical sensitizer that can remarkably promote a chemical reaction or physical phenomenon.

The "antibody" used herein refers to a protein molecule serving as a receptor for specifically recognizing an antigen, including an immunoglobulin molecule immunologically having reactivity with a specific antigen, and includes all of a polyclonal antibody, a monoclonal antibody, a full-length antibody, and an antibody fragment including an antigen-binding domain. A full-length antibody is a structure having two full-length light chains and two full-length heavy chains, and each light chain binds to a heavy chain by a disulfide bond. Whole antibodies include IgA, IgD, IgE, IgM and IgG, in which IgG is a subtype, including IgG1, IgG2, IgG3 and IgG4. The antibody fragment refers to a fragment having an antigen binding function, and includes Fab, Fab' F(ab')2, scFv and Fv. Fab is a structure having variable domains of a light chain and a heavy chain and the first constant domain (CH 1) of a heavy chain, and has one antigen-binding site. Fab' differs from Fab in that it has a hinge region including one or more cysteine residues at the C terminus of the heavy chain CH1 domain. An F(ab')2 antibody is produced by forming a disulfide bond between cysteine residues in the hinge region of Fab'. A variable fragment (Fv) refers to a minimal antibody fragment only having a heavy chain variable domain and a light chain variable domain. In double-chain Fv (dsFv), a heavy chain variable domain and a light chain variable domain are connected via a disulfide bond, and in a single-chain Fv (scFv), a heavy chain variable domain and a light chain variable domain are generally connected via a covalent bond using a peptide linker. Such antibody fragments may be obtained using a protease (for example, Fab may be obtained by cleaving the whole antibody with papain, and F(ab')2 fragment may be obtained by cleavage with pepsin), and preferably, may be manufactured through genetic recombination technology. In addition, the antibody of the present invention may be a natural antibody or recombinant antibody. A natural antibody refers to an antibody that is not genetically manipulated, and may have a remarkably low risk of immunogenicity that the genetically-manipulated antibody can have *in vivo.* A recombinant antibody refers to a genetically manipulated antibody, and may further have an antigen-binding force or a desired characteristic through genetic manipulation. Antibodies may include, for example, antibodies specifically binding to DEC205, CD206, DC-SIGN, DNGR1, CD11c, FcyR, PD-L1, PD-1, CD47, SIRP-α, Ly6G, IL-6, and Gr-1, and immune checkpoint inhibitors such as anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT.

The "drug for controlling immune system" used herein is a composition including a functional drug-toll-like receptor 7 or 8 agonist complex of the present invention as an active ingredient, and refers to a drug that activates immune cells, regulates the function of immune suppressor cells, and thus makes the immune system in the body work normally.

The "prevention" used herein refers to all actions that inhibit diseases such as cancer or delay the onset thereof by administration of the composition according to the present invention.

The "treatment" used herein refers to all actions that are involved in improving or beneficially changing symptoms of cancer by administration of the composition according to the present invention.

The "individual or subject" used herein refers to a target to which the composition of the present invention can be administered, and there is no limit to the target.

The "cancer" used herein is the generic term for various blood cancers, malignant solid tumors, etc. which can expand locally by infiltration and systemically by metastasis. Although not particularly limited, specific examples of cancer include colorectal cancer, adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and in situ carcinoma, Ewing's sarcoma, epidermoid carcinomas, giant cell tumors, glioblastoma multiforme, hairy-cell tumors, intestinal ganglioneuroma, hyperplastic corneal nerve tumors, islet cell carcinoma, Kaposi's sarcoma, leiomyoma, leukemias, lymphomas, malignant carcinoid, malignant melanomas, malignant hypercalcemia, marfanoid habitus tumors, medullary carcinoma, metastatic skin carcinoma, mucosal neuroma, myelodysplastic syndrome, myeloma, mycosis fungoides, neuroblastoma, osteosarcoma, osteogenic and other sarcoma, ovarian tumors, pheochromocytoma, polycythemia vera, primary brain tumors, small-cell lung tumors, squamous cell carcinoma of both ulcerating and papillary types, seminoma, soft tissue sarcoma, retinoblastoma, rhabdomyosarcoma, renal cell tumors or renal cell carcinoma, reticulum cell sarcoma, and Wilm's tumors. In addition, astrocytoma, gastrointestinal stromal tumors (GISTs), glioma or glioblastoma, hepatocellular carcinoma (HCC), and pancreatic neuroendocrine tumors are also included.

The "pharmaceutical composition" used herein may be prepared in the form of a capsule, tablet, granule, injection, ointment, powder, or drink, and the pharmaceutical composition may be intended for humans. The pharmaceutical composition may be, but is not limited to, formulated in the form of an oral formulation such as a powder, granules, a capsule, a tablet or an aqueous suspension, a preparation for external use, a suppository and a sterile injectable solution according to a conventional method. The pharmaceutical composition of the present invention may include a pharmaceutically acceptable carrier. The pharmaceutically acceptable carrier may be a binder, a glidant, a disintegrant, an excipient, a solubilizer, a dispersant, a stabilizer, a suspending agent, a colorant, or a flavoring agent for oral administration, and for injection, a mixture of a buffer, a preservative, a pain relief agent, a solubilizer, an isotonic agent, and a stabilizer may be used, and for topical administration, a base, an excipient, a lubricant, and a preservative may be used. The pharmaceutical composition of the present invention may be prepared in various forms by being mixed with the above-described pharmaceutically acceptable carriers. For example, for oral administration, the pharmaceutical composition of the present invention may be prepared in various dosage forms such as a tablet, a troche, a capsule, an elixir, a suspension, a syrup and a wafer, and for injectables, the pharmaceutical composition of the present invention may be prepared in a unit dose ampoule or multiple dose forms. In addition, the pharmaceutical composition of the present invention may be formulated as a solution, a suspension, a tablet, a capsule or a sustained-release preparation.

Meanwhile, the carrier, excipient, and diluent for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In addition, the carrier, excipient, and diluent for preparation may further include a filler, an anticoagulant, a lubricant, a wetting agent, a flavoring agent, an emulsifier, and a preservative.

The pharmaceutical composition according to the present invention is administered, but not limited to, orally, intravenously, intramuscularly, intraarterially, intramedullary, intrathecally, intracardially, transdermally, subcutaneously, intraperitoneally, intranasally, enterally, topically, sublingually, or rectally. Oral or parenteral administration is preferable. The term "parenteral" used herein means subcutaneous, intravenous, intramuscular, intraarticular, intrabursal, intrasternal, intrathecal, intralesional and intracranial injections or infusions. The pharmaceutical composition of the present invention may be administered in the form of a suppository for rectal administration.

The pharmaceutical composition of the present invention may vary according to various factors including the activity of a specific compound used, age, body weight, general health, sex, diet, administration time, administration route, excretion rate, drug formulation, and the severity of a specific disease to be prevented or treated, and the dose of the pharmaceutical composition may be selected by those of ordinary skill in the art according to a patient's condition and body weight, severity of a disease, a dosage form, an administration route and duration and may be administered daily at 0.0001 to 500 mg/kg or 0.001 to 500 mg/kg. The pharmaceutical composition of the present application may be administered once a day or several times in divided portions. The dose does not limit the scope of the present application in any way. The pharmaceutical composition according to the present invention may be formulated as a pill, a sugar-coated tablet, a capsule, a liquid, a gel, a syrup, a slurry or a suspension.

Hereinafter, to help in understanding the present invention, exemplary examples will be suggested. However, the following examples are merely provided to more easily understand the present invention, and not to limit the present invention.

### [Examples]

### Example 1: Synthesis of 2-methyl-1-(3-nitroquinolin-4- ylamino)propan-2-ol

2-Methyl-1-(3-nitroquinolin-4-ylamino)propan-2-ol (Compound 2) was synthesized using the method of Scheme 1 below. More specifically, 1-amino-2-methylpropan-2-ol (14 g) and tetraethylamine (9.6 g) were added to Compound 1 (30 g)-added dichloromethane (450 mL) at 10 to 20 °C and stirred for 2 hours, thereby preparing a mixture. Then, the mixture was concentrated by evaporating the solvent *in vacuo,* and then triturated with methyl tert-butyl ether (150 mL). The triturated mixture was separated using a filter and concentrated under low pressure, thereby obtaining Compound 2 (32 g, 85.2%, yellow solid). The structure of the obtained Compound 2 was verified using ¹H NMR.

¹H NMR (400 MHz, DMSO-d6): δ 9.91 (brs, 1H), 9.18 (s, 1H)), 8.46 (d, *J*= 8.0Hz, 1H), 7.83-7.92 (m, 2H), 7.56-7.60 (m, 1H), 5.15 (s, 1H), 3.86 (d, *J* = 4.8Hz, 2H), 1.15 (s, 6H).

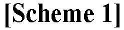

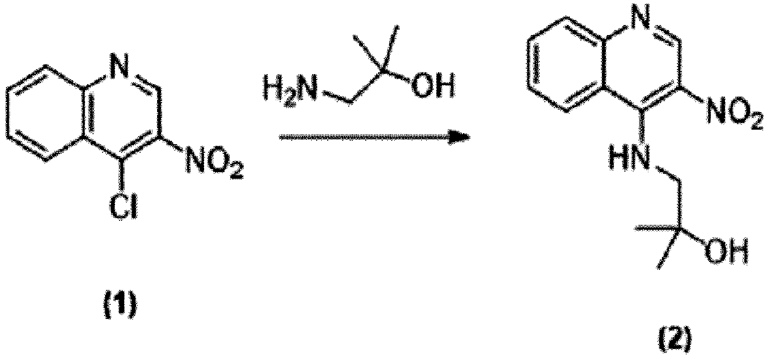

### Example 2: Synthesis of 1-(3-aminoquinolin-4-ylamino)-2-methylpropan-2-ol

1-(3-aminoquinolin-4-ylamino)-2-methylpropan-2-ol (Compound 3) was synthesized using the method of Scheme 2 below. In further detail, after mixing Compound 2 (32 g), methanol (500 mL) and Pd/C catalyst (3.2 g) in a reactor at 10 to 20 °C, the mixture was degassed and flushed three times using hydrogen. The hydrogen was vaporized to maintain 1 atm and the mixture was stirred at room temperature for 5 hours. Afterward, the mixture was triturated with methyl tert-butyl ether (100 mL). The triturated mixture was separated using a filter and concentrated at a low pressure, thereby obtaining Compound 3 (27 g, 95.4%, yellow solid). The structure of the obtained Compound 3 was verified using ¹H NMR.

¹H NMR (400 MHz, DMSO-d6): δ 8.37 (s, 1H)), 7.99-8.01 (m, 1H), 7.72-7.74 (m, 1H), 7.32-7.39 (m, 2H), 5.04 (s, 2H), 4.77 (brs, 1H), 4.67-4.70 (m, 1H), 4.12 (brs, 2H), 1.15 (s, 6H).

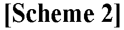

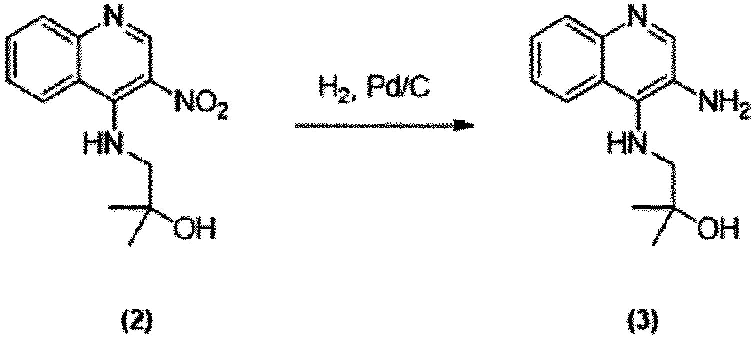

### Example 3: Synthesis of 1-(2-ethoxymethyl)-1H-imidazo[4,5-c]quinolin-1-yl)-2-methylpropan-2-ol

1-(2-ethoxymethyl)-1H-imidazo[4,5-c]quinolin-1-yl)-2-methylpropan-2-ol (Compound 4) was synthesized using the method of Scheme 3 below. In further detail, Compound 3 (27 g) and 2-ethoxyacetic acid (30 mL) were added to a reactor at 10 to 20 °C, and stirred at 120 to 130 °C for 5 hours. Afterward, the mixture was cooled to 20 to 25 °C, and then saturated sodium carbonate (150 mL) was added. Then, a reaction product was extracted using a mixed solution of dichloromethane and methanol (10/1, v/v). The extracted organic solution layer was washed with brine, and then moisture was removed over sodium sulfate (10 g). Then, the dehydrated organic solution layer was filtered using a filter and concentrated under low pressure, thereby obtaining Compound 4 (30 g, 85.8%, yellow gel). The structure of the obtained Compound 4 was verified using ¹H NMR.

¹HNMR (DMSO_d6 400 MHz): δ 9.18 (s, 1H)), 8.63 (d, *J=* 8.0Hz, 1H), 8.13 (dd, *J=* 1.6, 8.0Hz, 1H), 7.63-7.71 (m, 2H), 4.91 (s, 2H), 4.78 (brs, 2H), 3.54 (q, *J* = 6.8Hz, 2H), 1.10-1.18 (m, 9H).

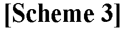

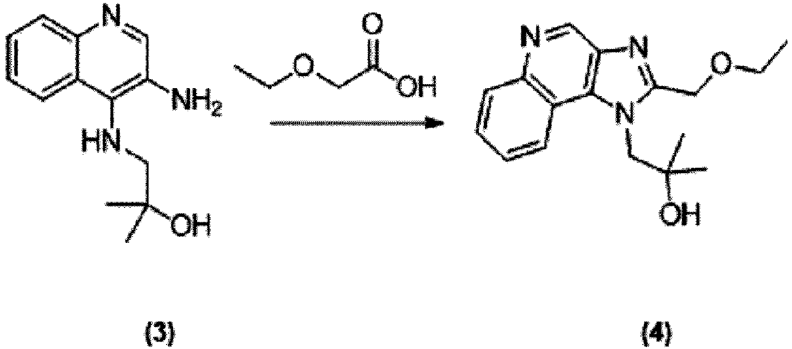

### Example 4: Synthesis of 2-(ethoxymethyl)-1-(2-hydroxy-2-methylpropyl)-1H-imidazo[4,5-c]quinolin 5-oxide

2-(ethoxymethyl)-1-(2-hydroxy-2-methylpropyl)-1H-imidazo[4,5-c]quinolin 5-oxide (Compound 5) was synthesized using the method of Scheme 4 below. In further detail, Compound 4 (30 g), dichloromethane (350 mL), and meta-chloroperoxybenzoic acid (26 g) were added to a reactor at 10 to 20 °C, and stirred at room temperature for 4 hours. Then, a saturated sodium carbonate solution (150 mL) and a sodium sulfate solution (150 mL) were added to the stirred mixture, and then a reaction product was extracted using a mixed solution of dichloromethane and methanol (10/1, v/v). The extracted organic solution layer was dehydrated over sodium sulfate (30 g), and filtered using a filter, followed by concentration under low pressure. Afterward, the concentrated reaction product was triturated using ethyl acetate (50 mL), separated using a filter, and then dried under low pressure, thereby obtaining Compound 5 (30 g, 94.9%, yellow solid). The structure of the obtained Compound 5 was verified using ¹H NMR.

¹HNMR (DMSO_d6 400 MHz): δ 9.04 (s, 1H)), 8.79 (d, *J=* 8.4Hz, 1H), 8.71 (d, *J=* 8.4Hz, 1H), 7.77-7.80 (m, 2H), 4.93 (s, 2H), 4.73 (brs, 2H), 3.54 (q, *J* = 6.8Hz, 2H), 1.12-1.18 (m, 9H).

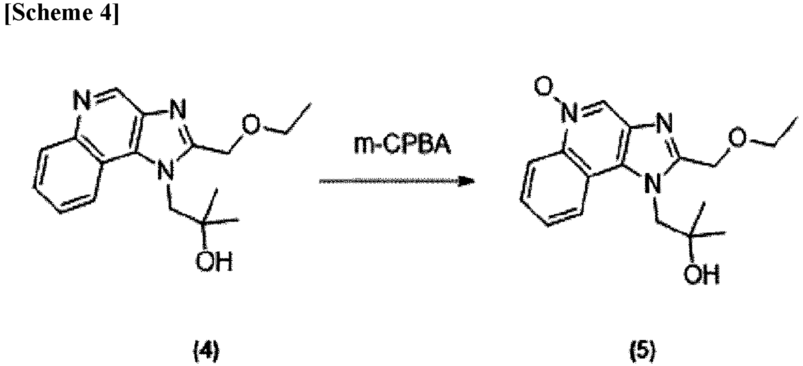

### Example 5: Synthesis of 1-(4-amino-2-ethoxymethyl)-1H-imidazo[4.5-c]quinolin-1-yl)-2-methylpropan-2-ol

1-(4-amino-2-ethoxymethyl)-1H-imidazo[4.5-c]quinolin-1-yl)-2-methylpropan-2-ol (Compound 6) was synthesized using the method of Scheme 5 below. In further detail, Compound 5 (30 g), DCM (600 mL), 4-methylbenzyl-1-sulfonyl chloride (18.2 g), and ammonia (NH₃.H₂O, 180 mL) were added to a reactor at 10 to 20 °C, and stirred at room temperature for 16 hours. Then, after adding distilled water to the stirred mixture, the mixture was separated using a mixed solution of dichloromethane and methanol (10/1, v/v). The separated organic solution layer was washed with brine and then dehydrated over anhydrous sodium sulfate (50 g). The dehydrated organic solution layer was filtered using a filter and then concentrated under low pressure, followed by triturating the concentrated reaction product using a mixed solution of methyl tert-butyl ether and methanol (15/1, v/v) for 30 minutes. Then, the resulting product was separated using a filter and dried under low pressure, thereby obtaining Compound 6 (18 g, 60%, yellow solid). The structure of the obtained Compound 6 was verified using ¹H NMR.

¹HNMR (DMSO_d6 400 MHz): δ 8.27 (d, *J=* 8.0Hz, 1H), 7.59 (d, *J=* 7.6Hz, 1H), 7.40 (t, *J* = 7.2Hz, 1H), 7.21 (t, *J* = 7.2Hz, 1H), 6.57 (brs, 2H), 4.89 (s, 2H), 4.68 (brs, 2H), 3.52 (q, *J=* 6.8Hz, 2H), 1.11-1.17 (m, 9H).

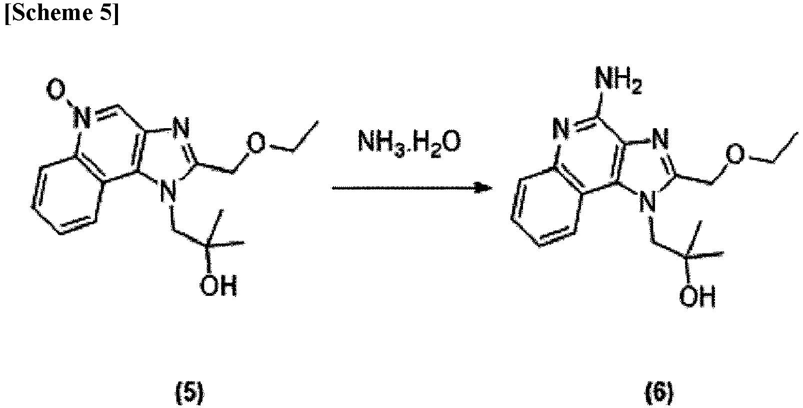

### Example 6: Synthesis of 10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethyl-1-(2-hydroxy-2-methylpropyl)-1H-imidazo[4,5-c]quinolin-4-yl carbamate

10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethyl)-1-(2-hydroxy-2-methylpropyl)-1H-imidazo[4,5-c]quinolin-4-yl carbamate (Compound 8) was synthesized using the method of Scheme 6 below. First, Compound 7 (TCI, 50 g, cholesterol chloroformate) was subjected to 250g-silica gel-filled column chromatography (0%~20% ethyl acetate in n-hexane), thereby obtaining pure Compound 7 (30 g). Then, Compound 6 (15 g) and dichloromethane (198.9 g) were added to a reactor at 10 to 20 °C, and the pure Compound 7 (30 g) and tetraethylamine (9.6 g) were sequentially added, followed by stirring at 20 to 25 °C for 16 hours. After adding water to the stirred mixture, dichloromethane was added to extract a reaction product. The extracted organic solution layer was washed with brine and dehydrated over anhydrous sodium sulfate (195 g). The dehydrated organic solution layer was filtered using a filter, and concentrated under low pressure. Then, the concentrated reaction product was triturated using a mixed solution of methyl tert-butyl ether and methanol (10/1, v/v). Afterward, Compound 8 (10.2 g, 55.1%, white solid) was obtained by separation using a filter and drying under low pressure. The structure of the obtained Compound 8 was verified using ¹H NMR. From the ¹H NMR result, it was confirmed that a complex in which resiquimod (R848) and cholesterol are connected via a carbamate bond was prepared.

¹H NMR (CDCl₃ 400 MHz): δ 8.13-8.19 (m, 2H), 7.59-7.63 (m, 1H)), 7.46-7.50 (m, 1H), 5.42-5.43 (m, 1H), 4.92 (brs, 2H), 4.72-4.80 (m, 3H), 3.68 (q, J = 6.8Hz, 2H), 3.24 (s, 1H), 2.51-2.59 (m, 1H), 2.36-2.47 (m, 1H), 1.96-2.11 (m, 3H), 1.81-1.95 (m, 2H), 1.45-1.75 (m, 9H), 1.02-1.35 (m, 27H), 0.94 (d, J = 6.4Hz, 3H), 0.89 (d, J = 6.4Hz, 6H), 0.71 (s, 3H).

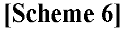

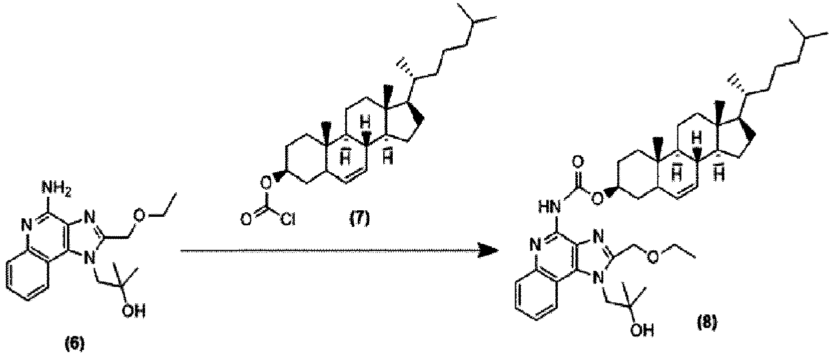

### Example 7: Synthesis of bis(2,5-dioxopyrrolidin-1-yl) 2,2'-disulfanedivlbis(ethane-2,1-divl) dicarbonate

Bis(2,5-dioxopyrrolidin-1-yl) 2,2' -disulfanediylbis(ethane-2,1-diyl) dicarbonate (Compound 9) was synthesized using the method of Scheme 7 below. First, Compound 7 (TCI, 70 g) was subjected to 350 g-silica gel-filled column chromatography (0%~20% ethyl acetate in n-hexane), thereby obtaining pure Compound 7 (40 g). Then, the pure Compound 7 (40 g) and dichloromethane (100 mL) were added to a reactor at 10 to 15 °C, and a bis(2-hydroxyethyl) disulfide-added dichloromethane (25 0mL) solution and pyridine (21 g) were sequentially added. The mixture was stirred at room temperature for 2 hours, and then distilled water (200 mL) was added. Subsequently, dichloromethane (150 mL) was added three times, thereby extracting a reaction product. The extracted organic solution layer was washed with brine and dehydrated over anhydrous sodium sulfate (20 g). The dehydrated reaction product was filtered using a filter, and then concentrated under low pressure. Afterward, Compound 9 (20 g, 39.6%, yellow gel) was obtained using column chromatography (silica gel, 300 g, 10%~30% ethyl acetate in n-hexane). The structure of the obtained Compound 9 was verified using ¹H NMR.

¹HNMR (CDCl₃ 400 MHz): δ 5.41-5.42 (m, 1H), 4.46-4.56 (m, 1H), 4.41 (t, *J* = 6.8Hz, 2H), 3.91 (t, *J=* 6.0Hz, 2H), 2.98 (t, *J* = 6.8Hz, 2H), 2.91 (t, *J=* 6.0Hz, 2H), 2.35-2.47 (m, 2H), 1.79-2.08 (m, 6H), 1.43-1.73 (m, 7H), 1.25-1.42 (m, 5H), 1.06-1.22 (m, 7H), 0.97-1.03 (m, 5H), 0.93 (d, *J* = 6.4Hz, 3H), 0.88 (dd, *J* = 1.6, 6.8Hz, 6H), 0.69 (s, 3H).

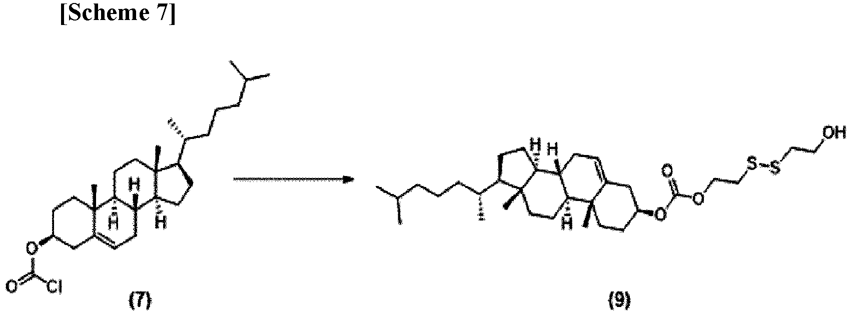

### Example 8: Synthesis of Compound 10

Compound 10 was synthesized using the method of Scheme 8 below. In further detail, Compound 9 (20 g) and dichloromethane (200 mL) were added to a reactor at 10 to 15 °C, and bis(2,5-dioxopyrrolidin-1-yl) carbonate (18 g) and tetraethylamine (10.7 g) were sequentially added. The mixture was stirred at room temperature for 3 hours, distilled water (300 mL) was added, and dichloromethane (150 mL) was then added three times to extract a reaction product. The extracted organic solution layer was washed with brine and dehydrated using anhydrous sodium sulfate (20 g). The dehydrated reaction product was filtered using a filter, and then the filtrate was concentrated under low pressure. Afterward, Compound 10 (18 g, 72%, yellow gel) was obtained using column chromatography (silica gel, 200 g, 5%~20% ethyl acetate in n-hexane). The structure of the obtained Compound 10 was verified using ¹H NMR.

¹HNMR (CDCl₃ 400 MHz): δ 5.39-5.40 (m, 1H), 4.57 (t, *J* = 6.8Hz, 2H), 4.43-4.52 (m, 1H), 4.37 (t, *J* = 6.4Hz, 2H), 2.96-3.03 (m, 4H), 2.84 (s, 4H), 2.34-2.44 (m, 2H), 1.78-2.04 (m, 5H), 1.42-1.73 (m, 7H), 1.22-1.40 (m, 5H), 1.06-1.20 (m, 7H), 0.95-1.04 (m, 5H), 0.91 (d, *J=* 6.0Hz, 3H), 0.86 (d, *J=* 6.4Hz, 6H), 0.67 (s, 3H).

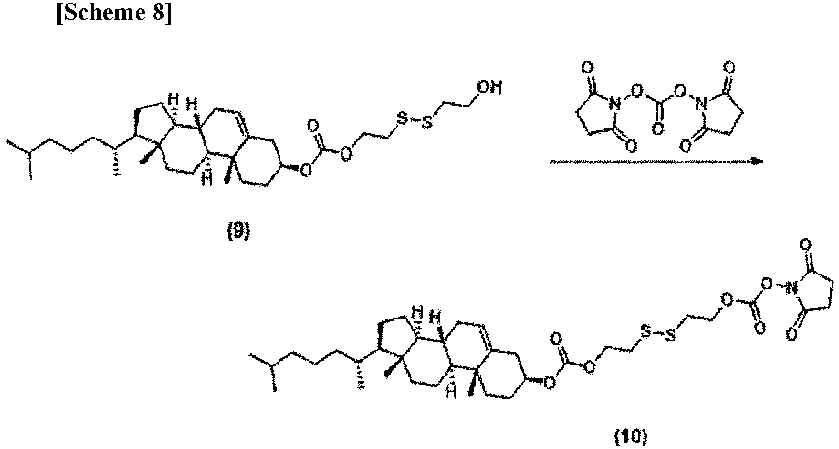

### Example 9: Synthesis of 2-((2-((10,13-diemthyl-17-(6-methylheptan-2-yl)-2,3,4,7,8,9,10,11,12,13,14,15,16,17-tetradecahydro-1H-cyclo[a]phenanthren-3-yloxy)cabonyloxy)ethyl)disulfanyl)ethyl 2-(ethoxymethyl)-1-(2-hydroxy-2-methylpropyl)-1H-imidazo[4,5-c]quinolin-4-yl carbamate

2-((2-((10,13-dimethyl-17-(6-methylheptan-2-yl)-2,3,4,7,8,9,10,11,12,13,14,15,16,17-tetradecahydro-1H-cyclo[a]phenanthren-3-yloxy)carbonyloxy)ethyl)disulfanyl)ethyl 2-(ethoxymethyl)-1-(2-hydroxy-2-methylpropyl)-1H-imidazo[4,5-c]quinolin-4-yl carbamate (Compound 11) was synthesized using the method of Scheme 9 below. In further detail, Compound 6 (15 g) and dichloromethane (198.9 g) were added to a reactor at 10 to 20 °C, and Compound 10 (40.5 g) and tetraethylamine (9.6 g) were sequentially added. The mixture was stirred at 20 to 25 °C for 16 hours, and then distilled water (225 mL) was added. Subsequently, dichloromethane (99.45 g) was added five times to extract a reaction product. The extracted organic solution layer was washed with brine and dehydrated over anhydrous sodium sulfate (195 g). Then, the dehydrated reaction product was filtered using a filter, and then the filtrate was concentrated under low pressure. Afterward, Compound 11 (10.8 g, 37.4%, white solid) was obtained using column chromatography (silica gel, 100 g, 10%~50% ethyl acetate in n-hexane). The structure of the obtained Compound 11 was verified using ¹H NMR. From the ¹H NMR result, it was confirmed that a complex in which R848 and cholesterol are crosslinked via disulfide was prepared.

¹H NMR (CDCl₃ 400 MHz): δ 8.15-8.17 (m, 2H), 7.60-7.64 (m, 1H)), 7.47-7.51 (m, 1H), 5.39-5.40 (m, 1H), 4.93 (s, 2H), 4.81 (s, 2H), 4.56 (t, *J=* 6.4Hz, 2H), 4.45-4.54 (m, 1H), 4.41 (t, *J=* 6.4Hz, 2H), 3.68 (q, *J* = 6.8Hz, 2H), 3.13 (s, 1H), 3.09 (t, *J=* 6.4Hz, 2H), 3.01 (t, *J=* 6.4Hz, 2H), 2.34-2.47 (m, 2H), 1.92-2.06 (m, 3H), 1.79-1.90 (m, 2H), 1.23-1.72 (m, 21H), 1.06-1.21 (m, 7H), 0.96-1.05 (m, 5H), 0.93 (d, *J=* 6.4Hz, 3H), 0.88 (dd, *J* = 1.6, 6.4Hz, 6H), 0.69 (s, 3H).

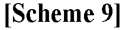

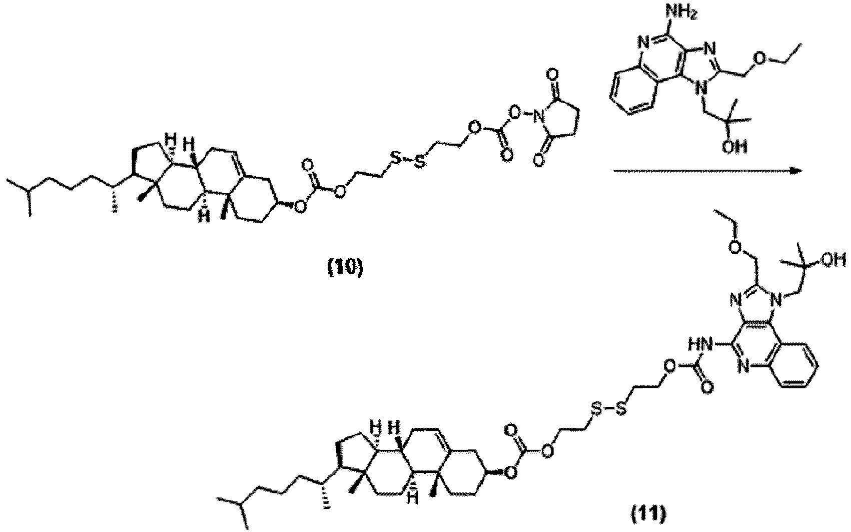

### Example 10: Synthesis of toll-like receptor 7 or 8 agonist-antibody complex

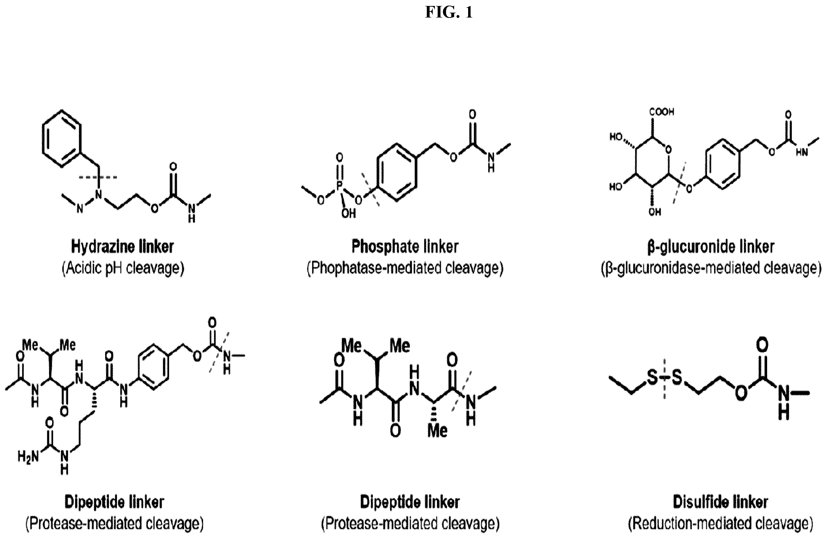

Various antibody-complexd toll-like receptor 7 or 8 agonists (an imidazoquinoloine-based agonist, an 8-hydroxyadenine-based agonist, a pteridone-based agonist, a 2-aminopyrimidine-based agonist, a benzoazepine-based agonist, a 7-thia-8-oxoguanosine-based agonist, etc.) may be prepared by the reaction between the amine group (NH₂), which is the active site of a toll-like receptor 7 or 8 agonist, and an antibody, an antibody fragment, an antibody derivative, by synthesizing a carbamate, disulfide, hydrazine, ester, peptide, azide, or β-glucuronide bond. The toll-like receptor 7 or 8 agonist and the antibody may be connected via a linker cleaved by various external or internal environmental stimuli. Representative linkers are shown in FIG. 1. Hereinafter, to confirm whether a complex in which the function of the toll-like receptor 7 or 8 agonist is temporarily inhibited, and is activated by cleavage under a specific condition is substantially formed, as an antibody-toll-like receptor 7 or 8 agonist complex, a complex in which resiquimod, which is a type of toll-like receptor 7 or 8 agonist, is bonded to an antibody by various bonds, was prepared.

### 10.1. Synthesis of toll-like receptor 7 or 8 agonist-antibody complex having bond cleaved by protease

To synthesize a complex in which an antibody and a toll-like receptor 7 or 8 agonist are connected via a peptide bond to be cleaved by a protease, the method of Scheme 10 or 11 below was used. As one specific example of Scheme 10, to synthesize a complex of a sulfhydryl group-containing antibody and resiquimod, having a bond cleaved by cathepsin B, resiquimod having a linker primarily connected to its active site was synthesized. In further detail, resiquimod (10 mg) and Mal-Val-Cit-PAB-PNP (47.2 mg) were added to 2 mL of N,N-dimethylformamide (DMF), and then dissolved. Afterward, N,N-diisopropylethylamine (DIPEA; 6.7 µL) and 1-hydroxybenzotriazole hydrate (1-HOBt; 315 µg) were added. Then, the mixture was prepared by stirring at room temperature for 16 hours. The prepared mixture was purified using thin-layer chromatography, thereby obtaining resiquimod (white powder) in which the function of the active site of the toll-like receptor 7 or 8 agonist is kinetically recovered by cathepsin-B.

In addition, to reduce the antibody, an anti-VEGFR2 antibody, which is a type of antibody, and tris(2-carboxyethyl)phosphine (TCEP) were added to a 1 mM EDTA solution (pH 7.4), and reacted for 1 hour at 37 °C. Then, after lowering the temperature to 22 °C, dehydroascorbic acid was added to adjust pH to 6.5 and reacted for 1 hour for reoxidation. Subsequently, the resiquimod complex in which the function of the active site of the toll-like receptor 7 or 8 agonist was kinetically recovered by cathepsin-B was treated with 1M Tris buffer to adjust pH to 7.4, mixed with an antibody, and reacted at 22 °C for 30 minutes, thereby preparing a complex, and unreacted materials were removed by gel electrophoresis using a PD-10 column.

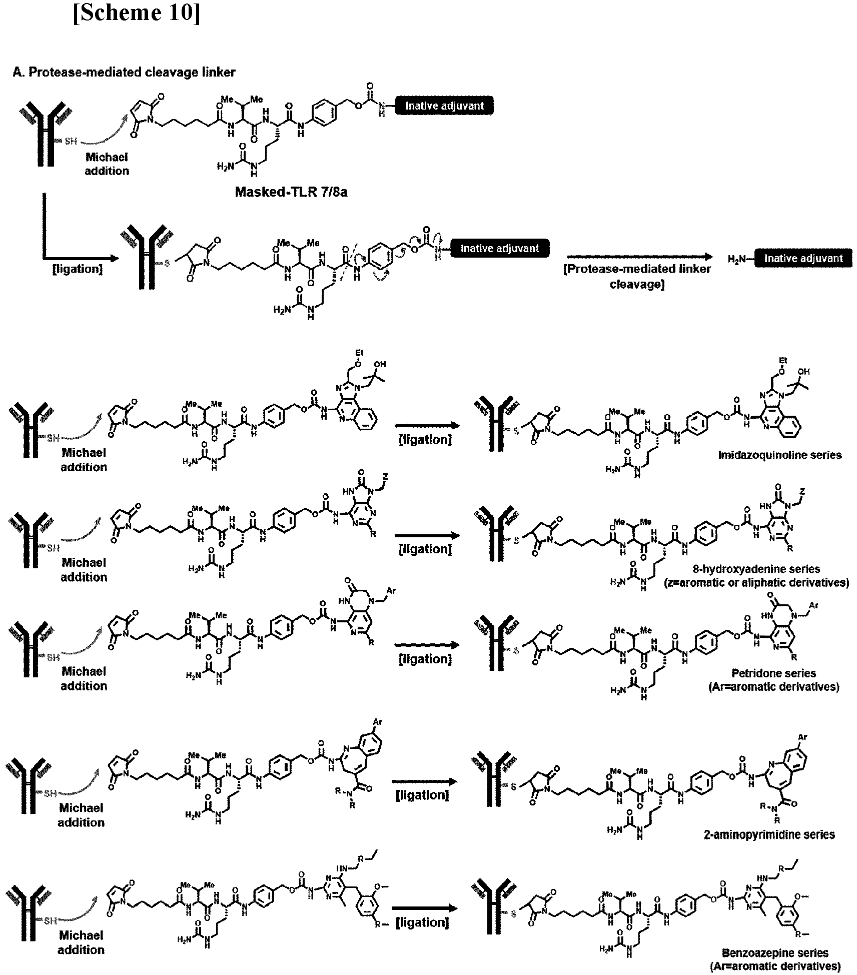

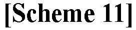

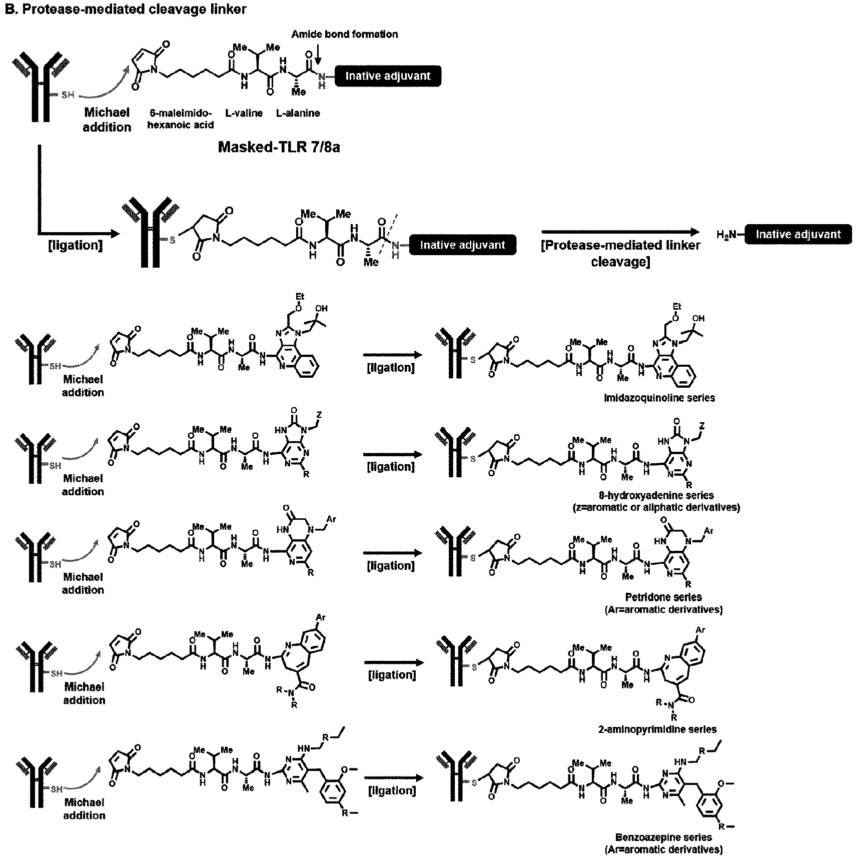

### 10.2. Synthesis of toll-like receptor 7 or 8 agonist-antibody complex having bond cleaved by β-glucuronidase

To synthesize a complex in which an antibody and a toll-like receptor 7 or 8 agonist are connected via a β-glucuronide bond to be cleaved by β-glucuronidase, the method of Scheme 12 below was used. First, to reduce the antibody, an anti-VEGFR2 antibody, which is a type of antibody, and tris(2-carboxyethyl)phosphine were added to a 1 mM EDTA solution (pH 7.4), and reacted for 1 hour at 37 °C. Then, after lowering the temperature to 22 °C, dehydroascorbic acid was added to adjust pH to 6.5 and reacted for 1 hour for reoxidation. Subsequently, a reaction between resiquimod, which is a type of β-glucuronine linker-connected (i.e., inactivated) toll-like receptor 7 or 8 agonist, and the sulfurhydryl group of a partially-reduced antibody was induced. In further detail, after adjusting pH to 7.4 using 1M Tris buffer, an antibody was mixed with resiquimod to which a β-glucuronine linker was connected, and reacted at 22 °C for 30 minutes, thereby preparing a complex, and unreacted materials were removed by gel electrophoresis using a PD-10 column.

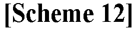

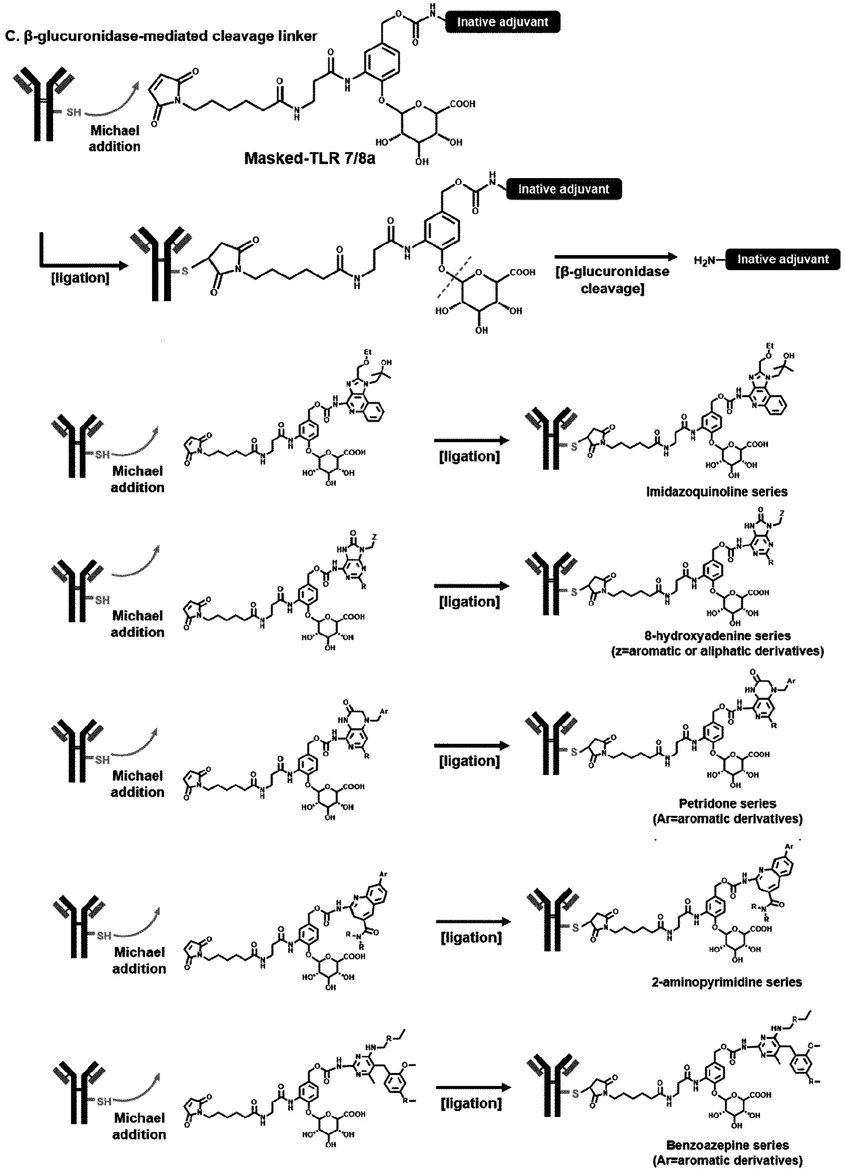

### 10.3. Synthesis of complex of toll-like receptor 7 or 8 agonist and antibody, having bond cleaved by reducing agent

To synthesize a complex in which an antibody and a toll-like receptor 7 or 8 agonist are connected via a disulfide bond to be cleaved by a reducing agent, the method of Scheme 13 below was used. In further detail, an anti-SIRPα antibody (90 mg), which is a type of antibody, was added to a solution prepared by adding resiquimod (31.4 mg), which is a type of toll-like receptor 7 or 8 agonist, and pyridine (100 µL) to dichloromethane (3 mL) and dissolved, and a maleimidocaproyl-carbonate-disulfide-NHS carbonate ester (90 mg)-added dichloromethane solution (1 mL) was then slowly added dropwise. Then, the mixture was prepared by stirring at 4 °C for 16 hours. Distilled water was added to the mixture to separate the aqueous layer and the dichloromethane layer, and then sodium sulfate was added to the separated dichloromethane layer and reacted for 16 hours to remove remaining water. Then, the remaining solution was purified using a silica gel column, thereby obtaining antibody-linked resiquimod as white powder.

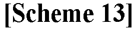

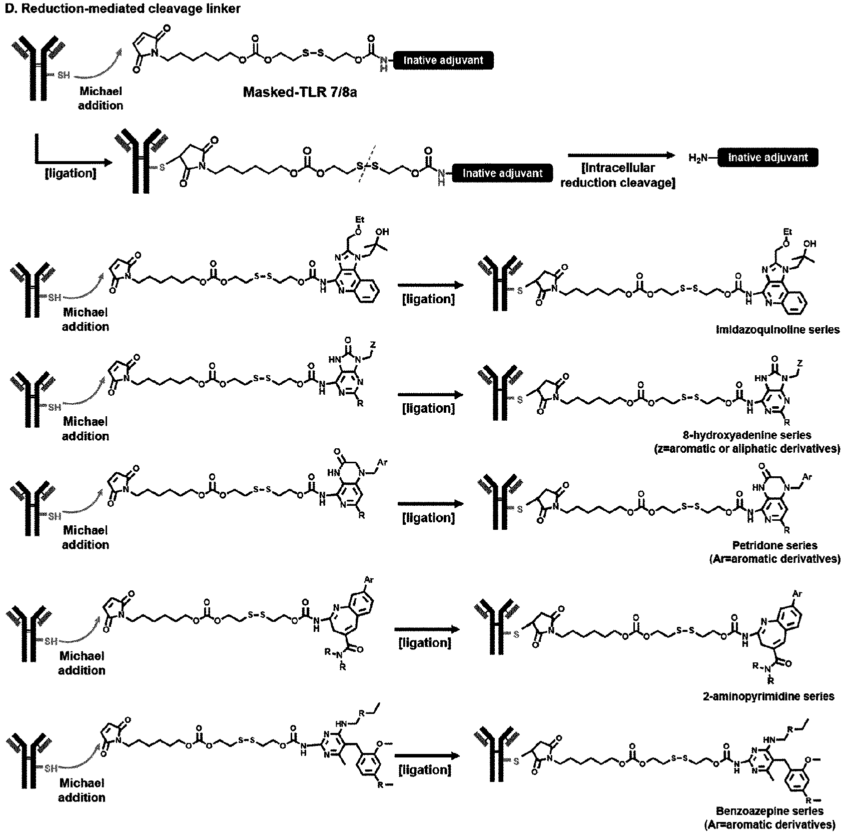

### 10.4. Synthesis of complex of toll-like receptor 7 or 8 agonist and antibody, having bond cleaved under acidic condition

To synthesize a complex in which an antibody and a toll-like receptor 7 or 8 agonist are connected via a hydrazine bond to be cleaved under an acidic condition in cells, the method of Scheme 14 below was used. In further detail, an anti-SIRPα antibody (10 µg), which is a type of antibody, was added to a solution prepared by adding resiquimod (31.4 mg), which is a type of toll-like receptor 7 or 8 agonist, and pyridine (100 µL) to dichloromethane (3 mL) and dissolved, and a maleimidocaproyl-carbonate-disulfide-NHS carbonate ester (90 mg)-added dichloromethane solution (1 mL) was then slowly added dropwise. Then, the mixture was prepared at 4 °C for 16 hours. Distilled water was added to the mixture to separate the aqueous layer and the dichloromethane layer, and then sodium sulfate was added to the separated dichloromethane layer and reacted for 16 hours to remove remaining water. Then, the remaining solution was purified using a silica gel column, thereby obtaining antibody-linked resiquimod as white powder.

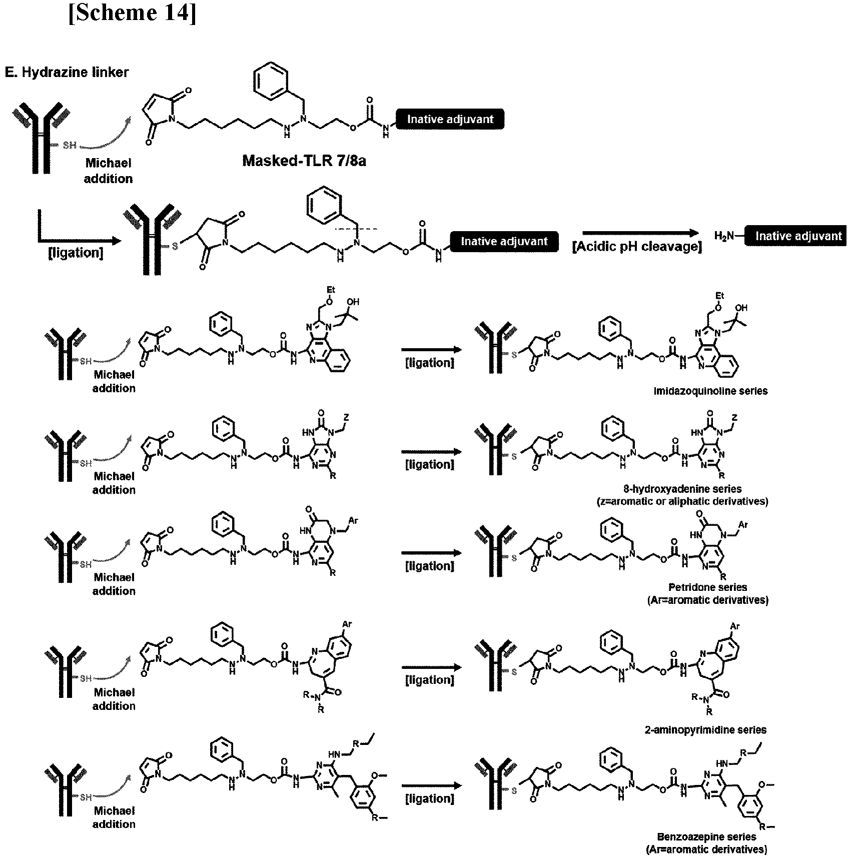

### Example 11: Synthesis of toll-like receptor 7 or 8 agonist-anticancer agent complex

Various anticancer agent-linked toll-like receptor 7 or 8 agonists (an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist) may be prepared by reactions between the amine (NH₂) group site, which is the active site of a toll-like receptor 7 or 8 agonist, and various anticancer agents capable of synthesizing a carbamate, disulfide, hydrazine, ester, peptide, azide, or β-glucuronidine bond. A toll-like receptor 7 or 8 agonist and an anticancer agent may be connected via a linker that is cleavable by various external or internal environmental stimuli. Hereinafter as an anticancer agent-toll-like receptor 7 or 8 agonist complex, to confirm whether a complex in which the function of the toll-like receptor 7 or 8 agonist is temporarily inhibited and activated by cleavage under a specific condition is substantially formed, complexs in which resiquimod, which is a type of toll-like receptor 7 or 8 agonist, is connected with an anticancer agent by various bonds were prepared.

### 11.1. Synthesis of complex of amine group-containing anticancer agent and toll-like receptor 7 or 8 agonist, having bond cleaved by reducing agent

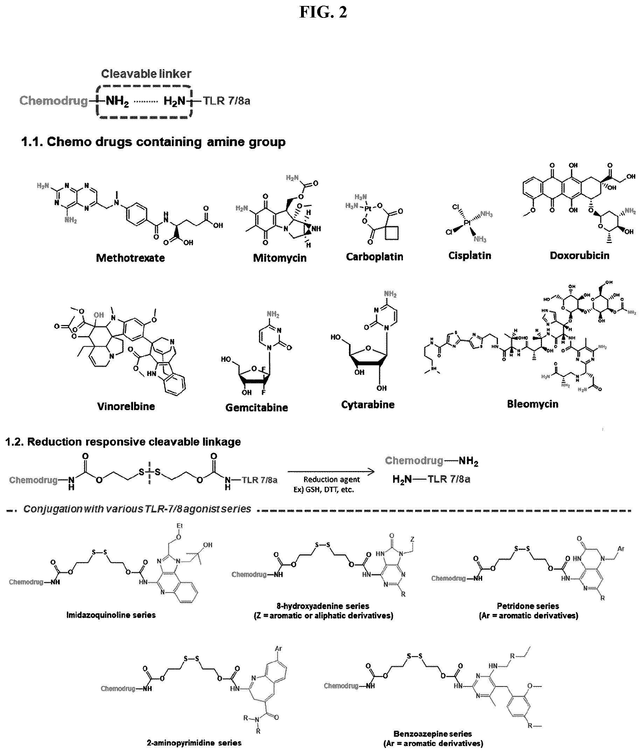

To synthesize a complex in which an amine group-containing anticancer agent (chemo-drug containing amine group) and a toll-like receptor 7 or 8 agonist are connected via a disulfide bond to be cleaved by a reducing agent, first, to reduce an amine group-containing anticancer agent, doxorubicin, which is a type of amine group-containing anticancer agent, and tris(2-carboxyethyl)phosphine (TCEP) were added to a 1 mM EDTA solution (pH 7.4), and reacted for 1 hour at 37 °C. Then, after lowering the temperature to 22 °C, dehydroascorbic acid was added to adjust pH to 6.5 and reacted for 1 hour for reoxidation. Afterward, a reaction between disulfide in a reduced state that was not oxidized, and a linker including resiquimod was induced. In further detail, after adjusting pH to 7.4 using 1M Tris buffer, the oxidized anticancer agent and resiquimod were mixed and reacted at 22 °C for 30 minutes, thereby preparing a complex, and then unreacted materials were removed by gel electrophoresis using a PD-10 column. Representative types of anticancer agents having an amine group, an example of a complex of an amine group-containing anticancer agent and a toll-like receptor 7 or 8 agonist, having a bond cleaved by a reducing agent, and a cleavage mechanism using a reducing agent are shown in FIG. 2.

### 11.2. Synthesis of complex of amine group-containing anticancer agent and toll-like receptor 7 or 8 agonist, having bond cleaved by hydrogen ion concentration (pH) in cells

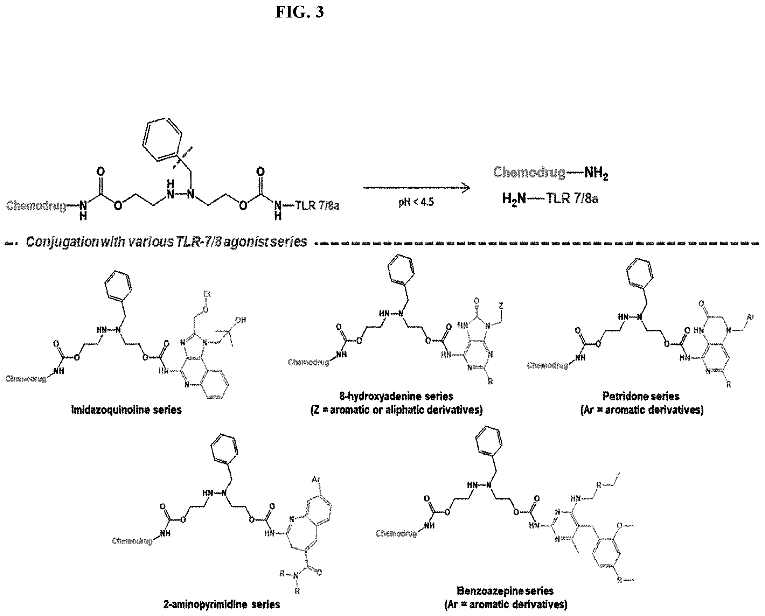

A complex in which an anticancer agent and a toll-like receptor 7 or 8 agonist are connected via a hydrazine bond to be cleaved under an acidic condition in cells was synthesized. In further detail, a dichloromethane solution (1 mL) to which aryl hydrazine (90 mg) and doxorubicin (31.4 mg), which is a type of amine group-containing anticancer agent, were added and dissolved, was slowly added dropwise to a solution prepared by adding resiquimod (31.4 mg), which is a type of toll-like receptor 7 or 8 agonist, and pyridine (100 µL) to dichloromethane (3 mL). Then, a mixture was prepared by stirring at 4 °C for 16 hours. Distilled water was added to the mixture to separate an aqueous layer and a dichloromethane layer, and sodium sulfate was added to the separated dichloromethane layer and reacted for 16 hours, thereby removing remaining water. Then, the remaining solution was purified using a silica gel column, thereby obtaining anticancer agent-linked resiquimod (white powder). Examples of complexs of an amine group-containing anticancer agent and a toll-like receptor 7 or 8 agonist, having a bond cleaved under an acidic condition, and a cleavage mechanism are shown in FIG. 3.

### 11.3. Synthesis of complex of anticancer agent and toll-like receptor 7 or 8 agonist, having bond cleaved by β-glucuronidase

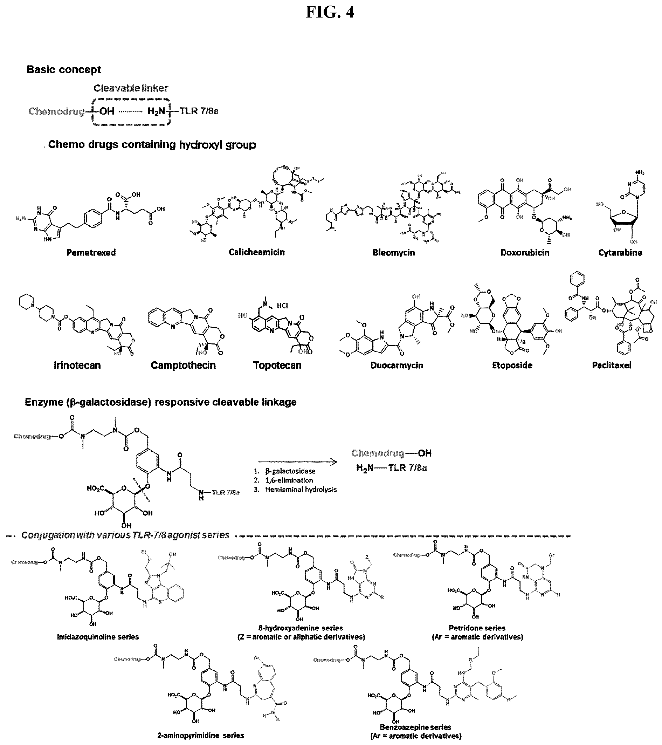

A complex in which an anticancer agent and a toll-like receptor 7 or 8 agonist are connected via a β-glucuronide bond to be cleaved by β-glucuronidase was synthesized. In further detail, a dichloromethane solution (1 mL) to which 4-nitrophenyl β-D-glucuronide (90 mg) and paclitaxel (31.4 mg), which is a type of anticancer agent having a hydroxyl group, were added and dissolved, was slowly added dropwise to a solution prepared by adding resiquimod (31.4 mg), which is a type of toll-like receptor 7 or 8 agonist, and pyridine (100 µL) to dichloromethane (3 mL). Then, a mixture was prepared by stirring at 4 °C for 16 hours. Distilled water was added to the mixture to separate an aqueous layer and a dichloromethane layer, and sodium sulfate was added to the separated dichloromethane layer and reacted for 16 hours, thereby removing the remaining water. Then, the remaining solution was purified using a silica gel column, thereby obtaining anticancer agent-linked resiquimod (white powder). Representative anticancer agents having a hydroxyl group and an example of a complex of an anticancer agent and a toll-like receptor 7 or 8 agonist, having a bond cleaved by β-glucuronidase, and cleavage mechanisms are shown in FIG. 4.

### 11.4. Synthesis of complex of hydroxyl group-containing anticancer agent and toll-like receptor 7 or 8 agonist, having bond cleaved by protease

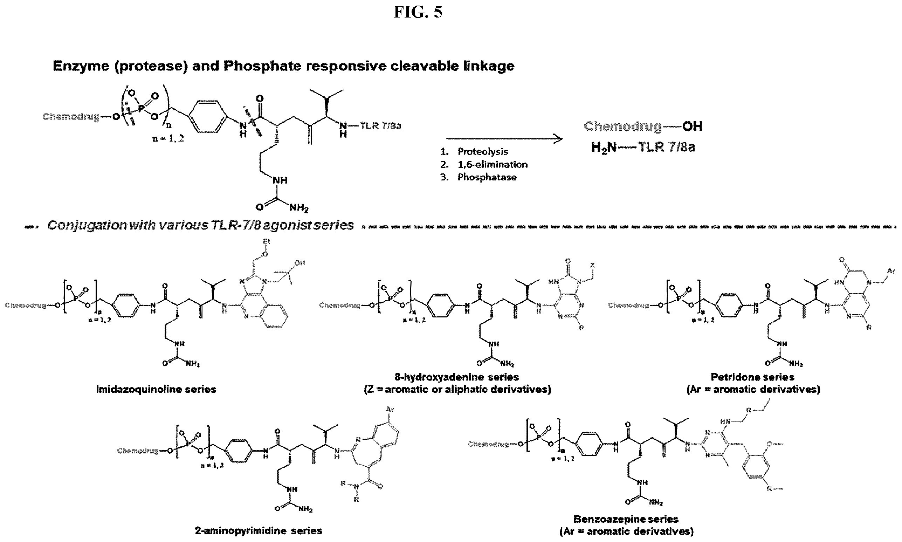

To synthesize a complex in which an anticancer agent and a toll-like receptor 7 or 8 agonist are connected via a peptide bond to be cleaved by a protease, first, to reduce an anticancer agent, paclitaxel, which is a type of hydroxyl group-containing anticancer agent, and tris(2-carboxyethyl)phosphine (TCEP) were added to a 1 mM EDTA solution (pH 7.4) and reacted for 1 hour at 37 °C. Then, after lowering the temperature to 22 °C, dehydroascorbic acid was added to adjust pH to 6.5 and reacted for 1 hour for reoxidation. The oxidized anticancer agent was reacted with phenyl phosphate (100 mg) to induce the reaction with resiquimod, which is a type of toll-like receptor 7 or 8 agonist. In further detail, after adjusting pH to 7.4 using 1M Tris buffer, the oxidized anticancer agent and resiquimod were mixed and reacted at 22 °C for 30 minutes, thereby preparing a complex, and unreacted materials were removed by gel electrophoresis using a PD-10 column. An example of a complex of a hydroxyl group-containing anticancer agent and a toll-like receptor 7 or 8 agonist, having a bond cleaved by a protease, and cleavage mechanisms are shown in FIG. 5.

### Example 12: Synthesis of complex of toll-like receptor 7 or 8 agonist and protein-based drug

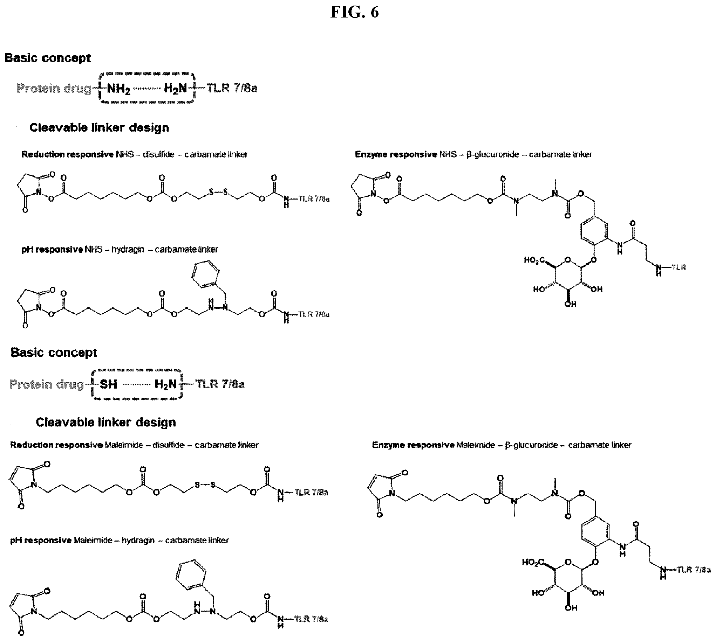

Various toll-like receptor 7 or 8 agonists (an imidazoquinoline-based agonist, an 8-hydroxyadenine-based agonist, a pteridone-based agonist, a 2-aminopyrimidine-based agonist, a benzoazepine-based agonist, and a 7-thia-8-oxoguanosine-based agonist) connected with a protein-based drug, that is, a functional drug consisting of a protein may be prepared by reactions between an amine group (NH₂) site, which is the active site of a toll-like receptor 7 or 8 agonist, and various protein-based drugs capable of synthesizing carbamate, disulfide, hydrazine, ester, peptide, azide, and β-glucuronidine bonds. The toll-like receptor 7 or 8 agonist and the protein-based drug may be connected via a linker cleaved by stimuli of various external or internal environments. Hereinafter as a complex of a protein-based drug and a toll-like receptor 7 or 8 agonist, to confirm whether a complex in which the function of a toll-like receptor 7 or 8 agonist is temporarily inhibited, and then activated by cleavage under a specific condition is substantially formed, complexs in which resiquimod, which is a type of toll-like receptor 7 or 8 agonist, is connected with a protein-based drug by various bonds were prepared. Representative linkers and linkage mechanisms for preparing a complex are shown in FIG. 6.

### 12.1. Synthesis of complex of protein-based drug and toll-like receptor 7 or 8 agonist, having bond cleaved by reducing agent through reaction of amine group

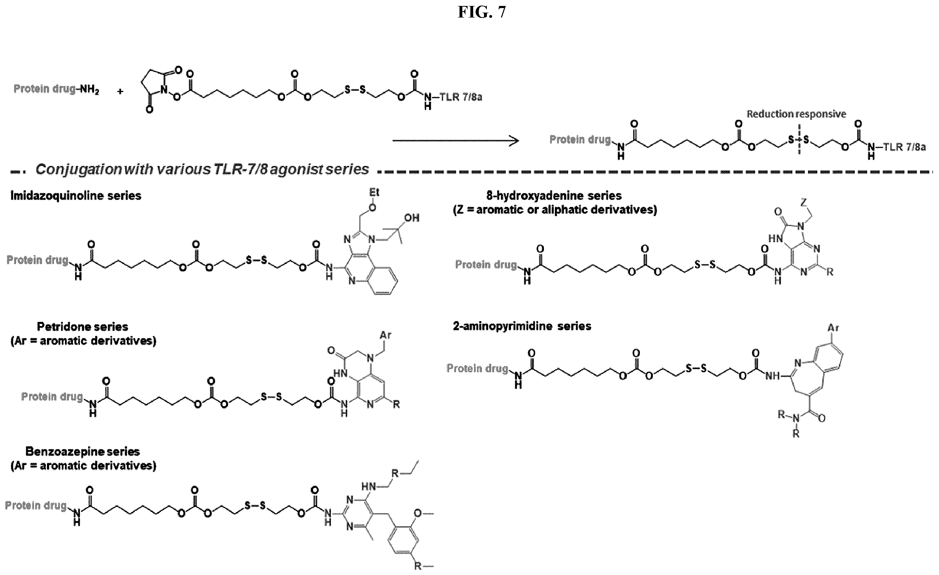

To synthesize a complex in which a protein-based drug and a toll-like receptor 7 or 8 agonist are connected via a disulfide bond to be cleaved by a reducing agent, an NHS ester reaction was used. In further detail, at 22 °C, dehydroascorbic acid was added to 3-mercaptopropyl-N-hydroxysuccinimide (NHS) ester to adjust pH to 6.5 and reacted for 1 hour, thereby preparing a linker. Afterward, after further increasing pH to 7.4 by the addition of 1M Tris buffer, a linker, the protein antigen ovalbumin (OVA), which is a type of protein-based drug, and resiquimod were mixed and reacted at 22 °C for 30 minutes, thereby preparing a complex. Unreacted materials were removed by gel electrophoresis using a PD-10 column. An example of a complex of a protein-based drug and a toll-like receptor 7 or 8 agonist, having a bond cleaved by a reducing agent, and cleavage mechanisms are shown in FIG. 7.

### 12.2. Synthesis of complex of protein-based drug and toll-like receptor 7 or 8 agonist, having bond cleaved by hydrogen ion concentration (pH) in cells through reaction of amine group

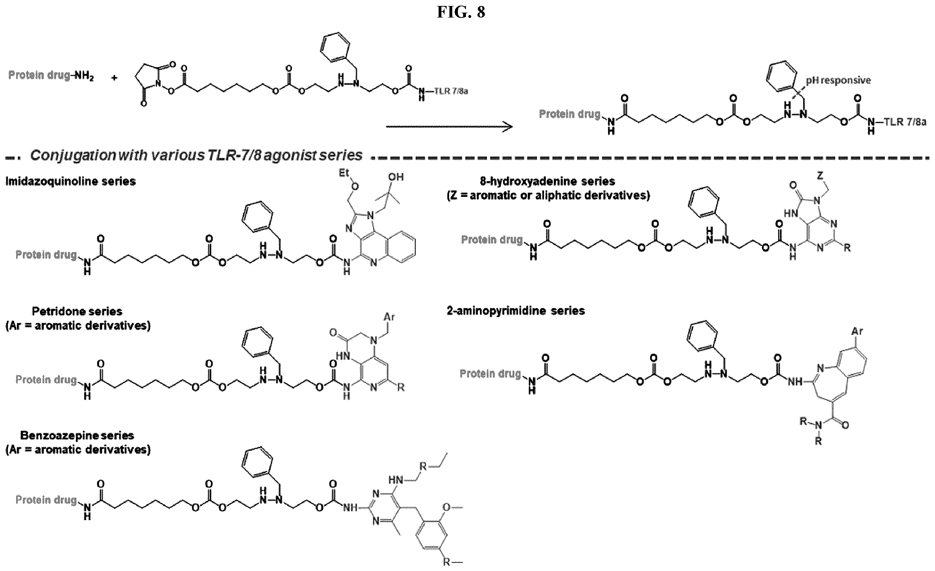

To synthesize a complex in which a protein-based drug is connected with a toll-like receptor 7 or 8 agonist by a hydrazine bond to be cleaved under a hydrogen ion concentration, i.e., an acidic condition in cells, an NHS ester reaction was used. In further detail, aryl hydrazine (90 mg)-added dichloromethane (1 mL) was slowly added dropwise to 3-mercaptopropyl-N-hydroxysuccinimide (NHS) ester- and pyridine (100 µL)-added dichloromethane (3 mL). Then, the protein antigen OVA (31.4 mg), which is a type of protein-based drug, and resiquimod (31.4mg) were added to the mixture and stirred at 4 °C for 16 hours, thereby synthesizing a complex. Then, the synthesized complex was purified using a silica gel column, thereby obtaining protein-based drug-linked resiquimod (white powder). An example of a complex of a protein-based drug and a toll-like receptor 7 or 8 agonist, having a bond cleaved by the concentration of hydrogen ions in cells, and cleavage mechanisms are shown in FIG. 8.

### 12.3. Synthesis of complex of protein-based drug and toll-like receptor 7 or 8 agonist, having bond cleaved by β-glucuronidase through reaction of amine group

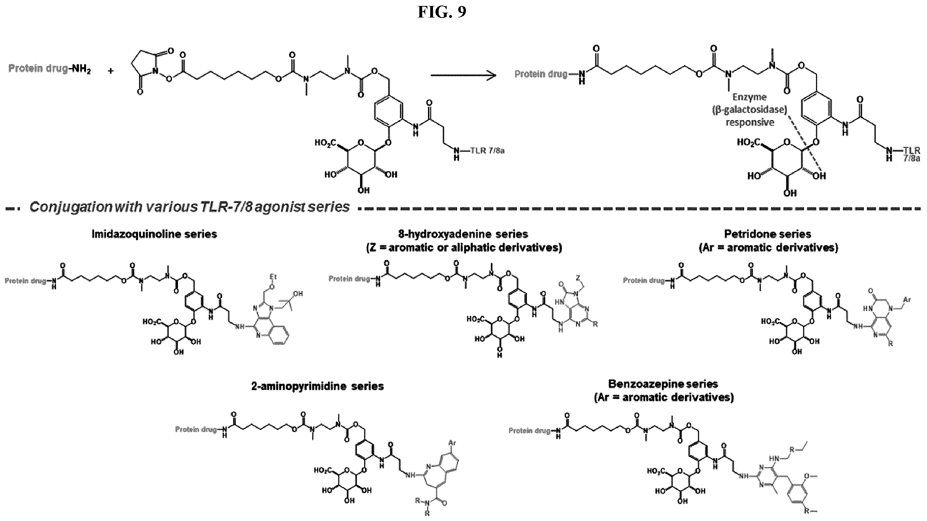

To synthesize a complex in which a protein-based drug and a toll-like receptor 7 or 8 agonist are connected via a β-glucuronide bond to be cleaved by β-glucuronidase, an NHS ester bond was used. In further detail, 4-nitrophenyl β-D-glucuronide (90 mg)-added dichloromethane (1mL) was slowly added dropwise to dichloromethane (3 mL) to which resiquimod (31.4 mg), 3-mercaptopropyl-N-hydroxysuccinimide (NHS) ester, and pyridine (100 µL) were added. Afterward, a mixture was prepared by stirring at 4 °C for 16 hours, the protein antigen OVA (31.4 mg), which is a type of protein-based drug, was added to the mixture and stirred at 4 °C for 16 hours, thereby synthesizing a complex. Then, after adjusting the temperature to room temperature, distilled water was added to separate an aqueous layer and a dichloromethane layer. Sodium sulfate was added to the separated dichloromethane layer and reacted for 16 hours to remove remaining water, and the remaining solution was purified using a silica gel column, thereby obtaining protein-based drug-linked resiquimod (white powder). An example of a complex of a protein-based drug and a toll-like receptor 7 or 8 agonist, having a bond cleaved by β-glucuronidase, and cleavage mechanisms are shown in FIG. 9.

### 12.4. Synthesis of complex of protein-based drug and toll-like receptor 7 or 8 agonist, having bond cleaved by reducing agent through reaction of sulfhydryl group

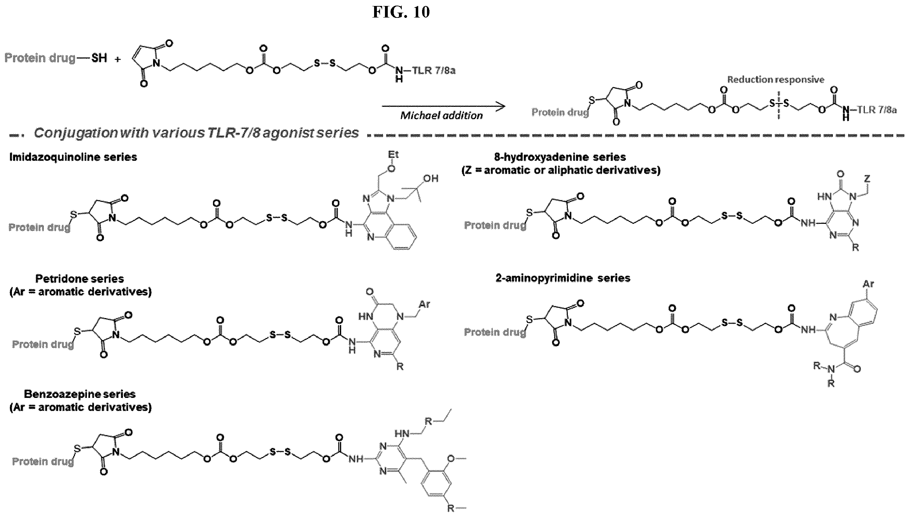

To synthesize a complex in which a protein-based drug and a toll-like receptor 7 or 8 agonist are connected via a disulfide bond to be cleaved by a reducing agent, a maleimide reaction was used. In further detail, dehydroascorbic acid was added to maleimide PEG at 22 °C to adjust pH to 6.5 and reacted for 1 hour thereby preparing a linker. Afterward, the reaction between the linker containing disulfide in a reduced state that was not oxidized, and the protein antigen OVA (31.4 mg), which is a type of protein-based drug, was induced. Subsequently, after adjusting pH to 9.0, the protein-based drug connected with the linker and resiquimod (31.4 mg) were mixed and reacted at 22 °C for 30 minutes. Then, unreacted materials were removed by gel electrophoresis using a PD-10 column. An example of a complex of a protein-based drug and a toll-like receptor 7 or 8 agonist, having a bond cleaved by a reducing agent through the reaction of a sulfhydryl group, and cleavage mechanisms are shown in FIG. 10.

### 12.5. Synthesis of complex of protein-based drug and toll-like receptor 7 or 8 agonist, having bond cleaved by hydrogen ion concentration (pH) in cells through reaction of sulfhydryl group

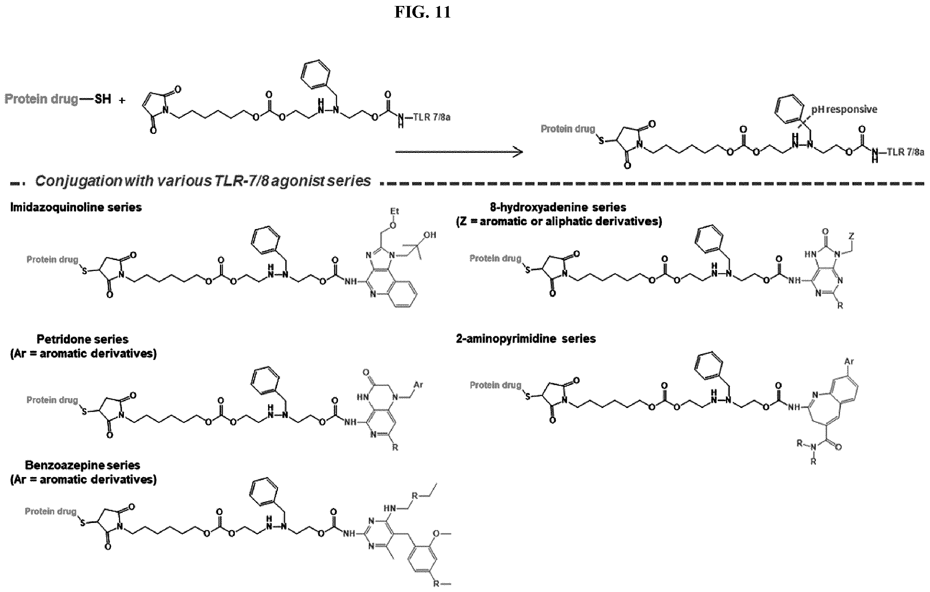

To synthesize a complex in which a protein-based drug and a toll-like receptor 7 or 8 agonist are connected via a hydrazine bond to be cleaved under a hydrogen ion concentration, i.e., acidic condition in cells, a maleimide reaction was used. In further detail, aryl hydrazine (90 mg)-added dichloromethane (1 mL) was slowly added dropwise to dichloromethane (3 mL) to which maleimide PEG and pyridine (100 µL) were added. Then, the protein antigen OVA (31.4 mg), which is a type of protein-based drug, and resiquimod (31.4 mg) were added to the mixture and stirred at 4 °C for 16 hours, thereby synthesizing a complex. Then, the synthesized complex was purified using a silica gel column, thereby obtaining protein-based drug-linked resiquimod (white powder). An example of a protein-based drug and a toll-like receptor 7 or 8 agonist, having a bond cleaved by a hydrogen ion concentration in cells, and cleavage mechanisms are shown in FIG. 11.

### 12.6. Synthesis of complex of protein-based drug and toll-like receptor 7 or 8 agonist, having bond cleaved by β-glucuronidase through reaction of sulfhydryl group

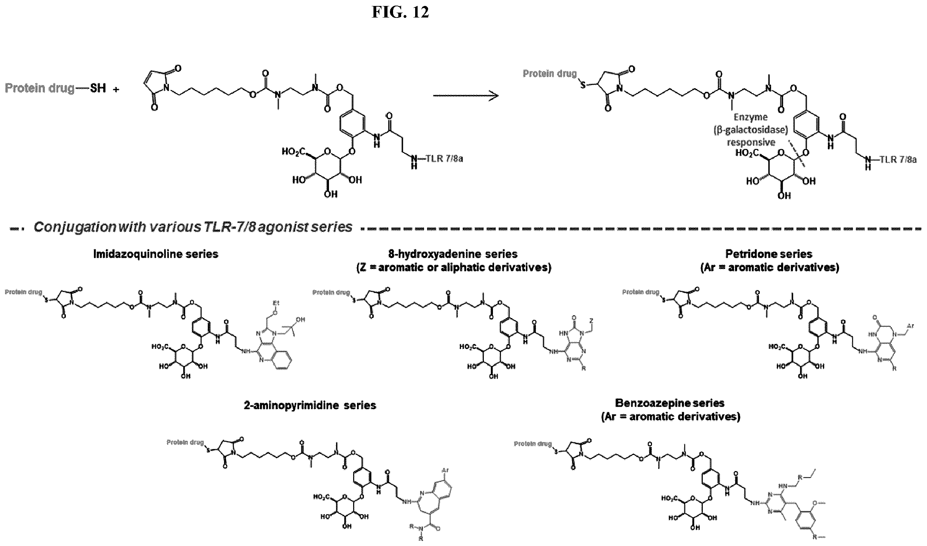

To synthesize a complex in which a protein-based drug and a toll-like receptor 7 or 8 agonist are connected via β-glucuronide to be cleaved by β-glucuronidase, a maleimide reaction was used. In further detail, 4-nitrophenyl β-D-glucuronide (90 mg)-added dichloromethane (1 mL) was slowly added dropwise to dichloromethane (3 mL) to which maleimide PEG and pyridine (100 µL) were added. Subsequently, after adjusting pH to 9.0, a mixture was prepared by stirring at 4 °C for 16 hours. The protein antigen OVA (31.4 mg), which is a type of protein-based drug, and resiquimod (31.4 mg) were added to the prepared mixture, and stirred at 4 °C for 16 hours, thereby synthesizing a complex. Then, after adjusting the temperature to room temperature, distilled water was added to separate an aqueous layer and a dichloromethane layer, sodium sulfate was added to the separated dichloromethane layer and reacted for 16 hours to remove remaining water. Then, the remaining solution was purified using a silica gel column, thereby obtaining protein-based drug-linked resiquimod (white powder). An example of a complex of a protein-based drug and a toll-like receptor 7 or 8 agonist, having a bond cleaved by β-glucuronidase, and cleavage mechanisms are shown in FIG. 12.

### Example 13: Synthesis of complex of toll-like receptor 7 or 8 agonist and sensitizer

### 13.1. Synthesis of complex of sensitizer and toll-like receptor 7 or 8 agonist, having bond cleaved by reducing agent

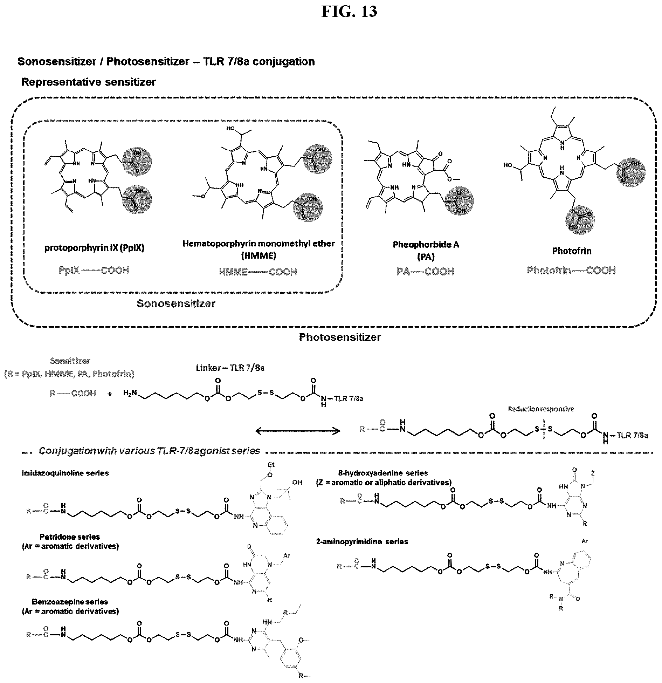

To synthesize a complex in which a sensitizer and a toll-like receptor 7 or 8 agonist are connected via a disulfide bond to be cleaved by a reducing agent, first, a sensitizer and tris(2-carboxyethyl)phosphine were added to a 1 mM EDTA solution (pH 7.4) and reacted for 1 hour at 37 °C to reduce Photofrin, which is a type of photosensitizer. After lowering the temperature to 22 °C, dehydroascorbic acid was then added to adjust pH to 6.5 and reacted for 1 hour for reoxidation. Afterward, the reaction between Photofrin in a reduced state that was not oxidized, and resiquimod was induced. In further detail, after adjusting pH to 7.4 using 1M Tris buffer, the oxidized sensitizer and resiquimod were mixed and reacted at 22 °C for 30 minutes, thereby preparing a complex, and unreacted materials were removed by gel electrophoresis using a PD-10 column. Representative types of sensitizers, an example of a complex of a sensitizer and a toll-like receptor 7 or 8 agonist, having a bond cleaved by a reducing agent, and cleavage mechanisms are shown in FIG. 13.

### 13.2. Synthesis of complex of carboxyl group-containing sensitizer and toll-like receptor 7 or 8 agonist, having bond cleaved by hydrogen ion concentration (pH) in cells

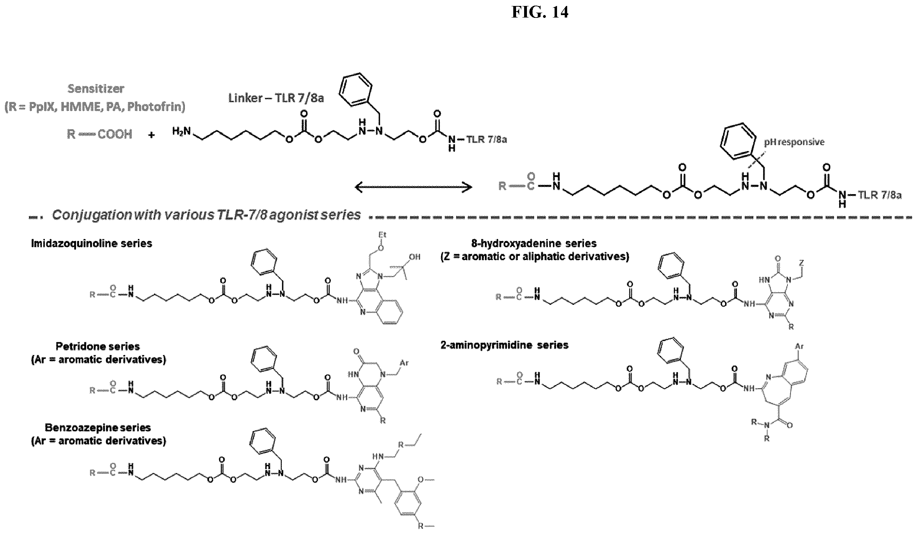

A complex in which a sensitizer and a toll-like receptor 7 or 8 agonist are connected via a hydrazine bond to be cleaved by a hydrogen ion concentration, i.e., under an acidic condition in cells, was synthesized. In further detail, an aryl hydrazine (90 mg)-added dichloromethane (1 mL) solution was slowly added dropwise to a solution prepared by adding and dissolving resiquimod (31.4 mg), which is a type of toll-like receptor 7 or 8 agonist, and pyridine (100 µL) to dichloromethane (3 mL). Subsequently, a mixture was prepared by stirring at 4 °C for 16 hours. Photofrin, which is a type of photosensitizer, was added to the mixture and reacted at 22 °C for 30 minutes, thereby preparing a complex, distilled water was then added to separate an aqueous layer and a dichloromethane layer, and sodium sulfate was added to the separated dichloromethane layer and reacted for 16 hours to remove remaining water. Then, the remaining solution was purified using a silica gel column, thereby obtaining sensitizer-linked resiquimod (white powder). An example of complex of a carboxyl group-containing sensitizer and a toll-like receptor 7 or 8 agonist, having a bond cleaved under an acidic condition, and cleavage mechanisms are shown in FIG. 14.

### 13.3. Synthesis of complex of carboxyl group-containing sensitizer and toll-like receptor 7 or 8 agonist, having bond cleaved by β-glucuronidase

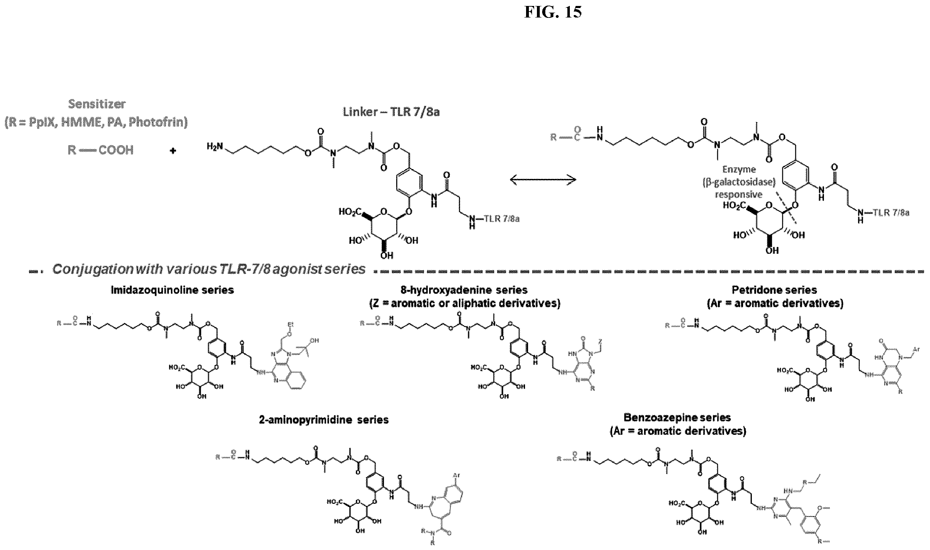

A sensitizer and a toll-like receptor 7 or 8 agonist are connected via a β-glucuronide bond to be cleaved by β-glucuronidase was synthesized. In further detail, a nitrophenyl β-gluconide (90 mg)-added dichloromethane (1 mL) solution was slowly added dropwise to a solution prepared by adding and dissolving resiquimod (31.4 mg), which is a type of toll-like receptor 7 or 8 agonist, and pyridine (100 µL) to dichloromethane (3 mL). Subsequently, a mixture was prepared by stirring at 4 °C for 16 hours. Photofrin, which is a type of photosensitizer, was added to the mixture and reacted at 22 °C for 30 minutes, thereby preparing a complex, distilled water was then added to separate an aqueous layer and a dichloromethane layer, and sodium sulfate was added to the separated dichloromethane layer and reacted for 16 hours to remove remaining water. Then, the remaining solution was purified using a silica gel column, thereby obtaining sensitizer-linked resiquimod (white powder). An example of complex of a carboxyl group-containing sensitizer and a toll-like receptor 7 or 8 agonist, having a bond cleaved by β-glucuronidase, and cleavage mechanisms are shown in FIG. 15.

### Example 14: Complex of carboxyl group-containing antibody and resiquimod, having bond cleaved by hydrolysis

### 14.1. Synthesis of carboxyl group-containing antibody and resiquimod, having bond cleaved by hydrolysis

To synthesize a complex of a carboxyl group-containing antibody and resiquimod, having a bond cleaved by hydrolysis, first, a toll-like receptor 7 or 8 agonist in which the function of an active site is kinetically recovered by hydrolysis was synthesized. In further detail, resiquimod (10 mg) and maleimide-dPEG₄-NHS ester (19.6 mg) was added to dichloromethane (DCM; 2 mL), and then triethylamine (8.8 µL) was added. Subsequently, a mixture was prepared by stirring at room temperature for 16 hours, and the mixture was purified using thin-layer chromatography, thereby obtaining resiquimod in which the function of the active site is kinetically recovered by hydrolysis (white powder). Afterward, to bond resiquimod with an antibody, an anti-VEGFR2 antibody (4 mg) was dispersed in 1 mM diethylenetriamine (DTPA)-containing 1M borate buffer. In addition, 120 µM tris(2-carboxyethyl)phosphine (TCEP) was added and stirred at 37 °C for 1 hour for reduction. In addition, the reduced antibody and resiquimod (210 µg) were mixed and stirred at 4 °C for 1 hour. Afterward, the antibody-resiquimod complex (AAC-001) was purified by centrifugation using a Zeba^{™} desalting column at 1,000×g for 2 minutes.

### 14.2. Confirmation of anticancer effect of complex of carboxyl group-containing antibody and resiquimod, having bond cleaved by hydrolysis

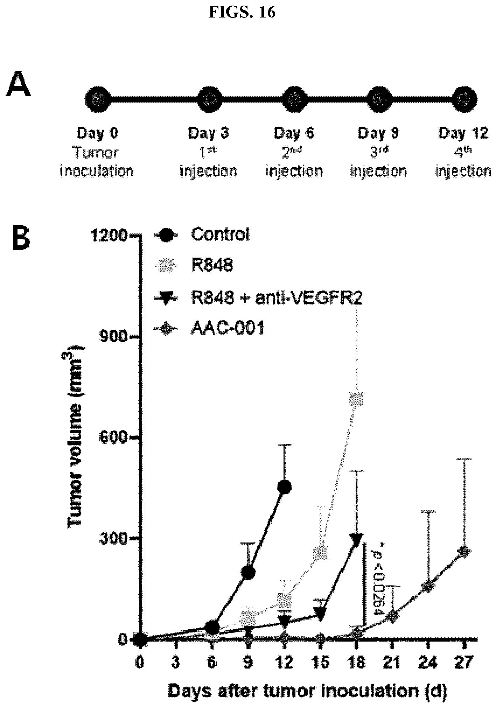

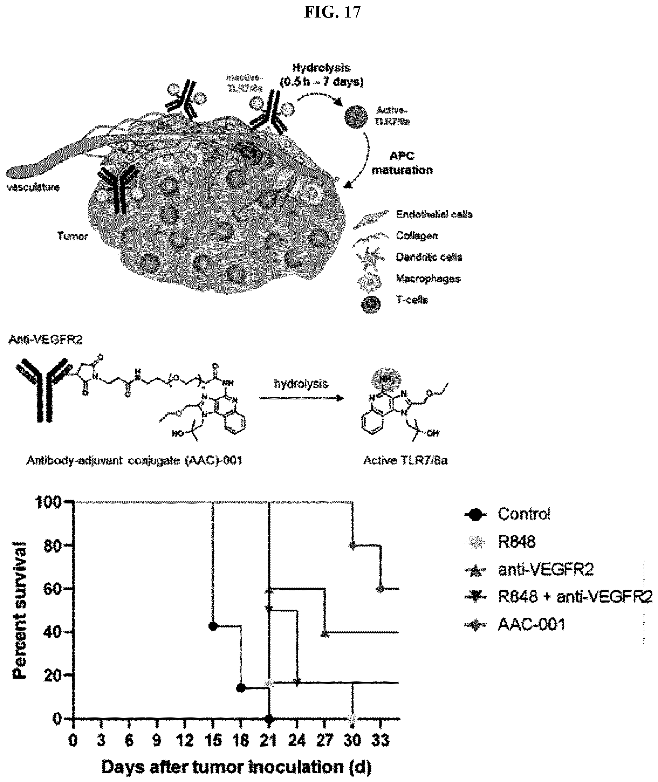

To confirm the anticancer effect of an antibody-resiquimod complex (AAC-001) synthesized in the same manner as in Example 14.1, the anticancer effect was evaluated using B16OVA melanoma. First, 5×10⁵ of B16OVA cancer cells were administered into the dorsal area of a mouse via subcutaneous injection, three days later, a drug was directly injected into cancer tissue 4 times at intervals of three days. For drugs, resiquimod, a mixture of resiquimod and anti-VEGFR2, and an anti-VEGFR2 antibody-resiquimod complex (AAC-001) having a bond cleaved by hydrolysis were used. Afterward, the degree of cancer growth was confirmed by measuring a cancer size, and a survival rate was also measured. Afterward, all experiments were repeated in at least triplicate, and the result is expressed as mean ± SD. Statistical significance was confirmed by the Student's t-test, and when P < 0.05, it was determined to be statistically significant. The results are shown in FIGS. 16 and 17.

As shown in FIGS. 16 and 17, it was confirmed that the AAC-001 complex may not only remarkably inhibit cancer growth but also increase a survival rate, compared to both single administration of resiquimod (R848) and co-administration of anti-VEGFR2 and resiquimod (R848+anti-VEGFR2).

### 14.3. Confirmation of blood inflammatory cytokine level of carboxyl group-containing antibody-resiquimod complex having bond cleaved by hydrolysis

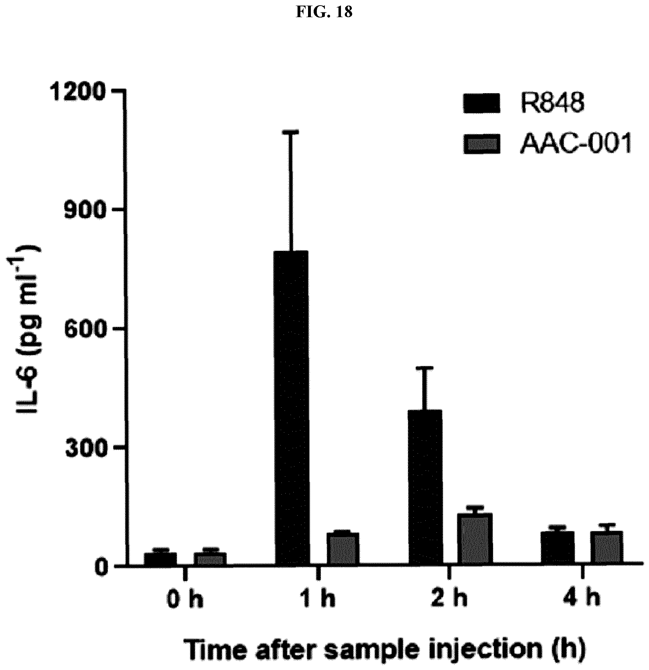

To confirm the effect of the antibody-resiquimod complex (AAC-001) synthesized in the same manner as in Example 14.1 on the secretion of inflammatory cytokine in blood, after intravenously injecting the AAC-001 complex or resiquimod into a mouse, blood samples were collected at 0, 1, 2, and 4 hours. The collected blood sample was centrifuged at 10,000×g for 20 minutes to separate serum, and the concentration of IL-6, which is a representative inflammatory cytokine, present in serum was measured using the Invitrogen mouse IL-6 ELISA kit. The result is shown in FIG. 18.

As shown in FIG. 18, it was confirmed that, as soon as resiquimod is injected into the body, it promotes the secretion of inflammatory cytokines and induces a cytokine storm, but the AAC-001 complex does not show a change in inflammatory cytokine level.

From the above results, it was confirmed that a complex of an antibody and a toll-like receptor 7 or 8 agonist according to the present invention, i.e., a complex in which an antibody binds to the active site of a toll-like receptor 7 or 8 agonist by a cleavable linker exhibits low side effects and a remarkably increased therapeutic effect, compared with simple co-treatment, because a functional drug such as an antibody works at the beginning of administration, and a toll-like receptor 7 or 8 agonist is temporarily inactive and then is activated by cleaving a linker with a hydrolase in a tumor microenvironment or target cells, and thus secondarily serves to activate the immune system.

### Example 15: Complex of carboxyl group-containing antibody and resiquimod, having bond cleaved by cathepsin-B

### 15.1. Synthesis of carboxyl group-containing antibody-resiquimod complex having bond cleaved by cathepsin-B

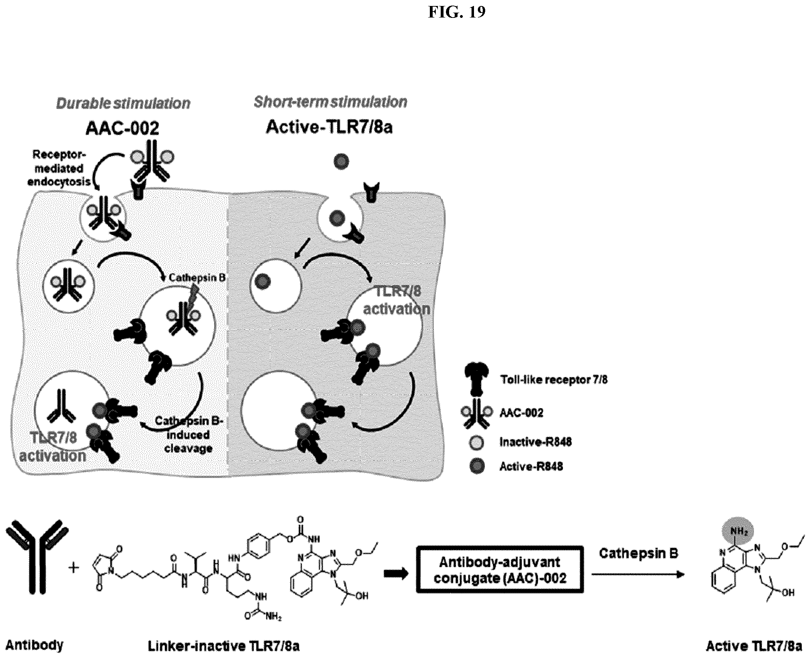

To synthesize a complex of a carboxyl group-containing antibody and resiquimod, having a bond cleaved by cathepsin B, first, resiquimod to which a linker binds to the active site was synthesized. In further detail, resiquimod (10 mg) and Mal-Val-Cit-PAB-PNP (47.2 mg) were added to 2 mL of N,N-dimethylformamide (DMF), and then dissolved. Afterward, N,N-diisopropylethylamine (DIPEA; 6.7 µL) and 1-hydroxybenzotriazole hydrate (1-HOBt; 315 µg) were added. Then, a mixture was prepared by stirring at room temperature for 16 hours. The prepared mixture was purified using thin-layer chromatography, thereby obtaining resiquimod in which the function of the active site of a toll-like receptor 7 or 8 agonist is kinetically recovered by cathepsin-B (white powder). Afterward, an antibody-resiquimod complex (AAC-002) having a bond cleaved by cathepsin-B was synthesized. To this end, after dispersing an anti-SIRPα antibody (4 mg) in 1M borate buffer to which 1 mM diethylenetriamine (DTPA) was added, 150 µM tris(2-carboxyethyl)phosphine (TCEP) was added and stirred at 37 °C for 1 hour to reduce the antibody. Subsequently, partially-reduced antibodies and resiquimod in which the function of its active site is kinetically recovered (480 µg) were mixed and stirred at 4 °C for 1 hour, thereby synthesizing complexs. Then, the resulting complexs were centrifuged at 1,000×g for 2 minutes using a Zeba^{™} desalting column, thereby separating only an antibody-resiquimod complex, AAC-002. The mechanism of the AAC-002 complex is shown in FIG. 19.

### 15.2. Confirmation of blood inflammatory cytokine level of carboxyl group-containing antibody and resiquimod complex having bond cleaved by cathepsin-B

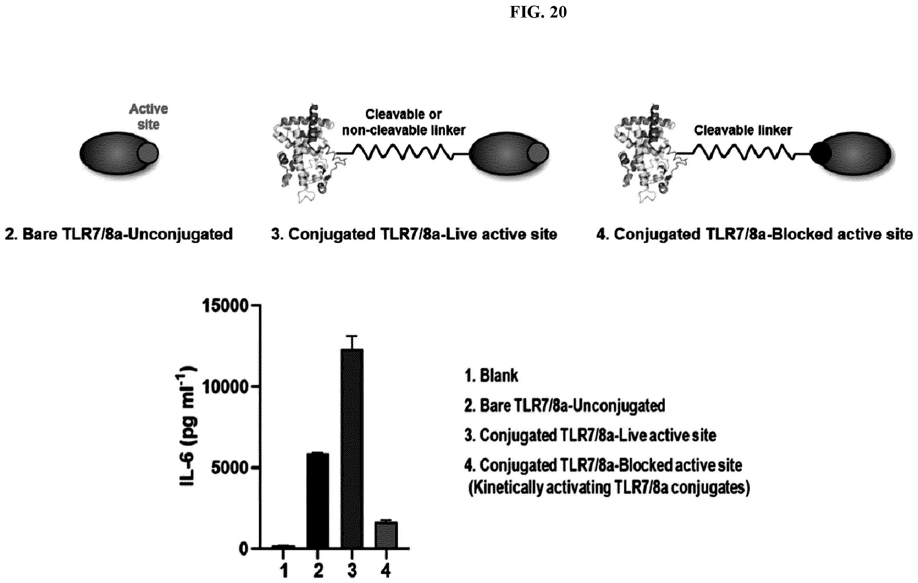

To confirm the effect of the antibody-resiquimod complex (AAC-002) synthesized in the same manner as in Example 15.1 on the secretion of an inflammatory cytokine in blood, two hours after the AAC-002 complex (conjugated TLR7/8a-Blocked active site) was intravenously injected into a mouse, blood samples were collected. The collected blood sample was centrifuged at 10,000×g for 20 minutes to separate serum, and the concentration of IL-6, which is a representative inflammatory cytokine, present in serum was measured using the Invitrogen mouse IL-6 ELISAkit. As controls, a phosphate-buffered saline-injected group (Blank), a resiquimod only-injected group (Bare TLR7/8a-Uncomplexd), and a group into which an antibody-resiquimod complex in which the antibody binds to a site other than the active site, is injected (conjugated TLR7/8a-Live active site) were used for comparison. The result is shown in FIG. 20.

As shown in FIG. 20, when resiquimod was administered alone, or in the case of the antibody-resiquimod complex in which the antibody binds to a site other than the active site, it was confirmed that, due to the active site, unnecessary stimulation is induced, thereby remarkably increasing an inflammatory cytokine level in blood at the beginning of injection. On the other hand, in the case of the complex in which the antibody binds to the active site, it was confirmed that the inflammatory cytokine level is not changed.

### Example 16: Protein antigen-resiquimod complex having bond cleaved by cathepsin-B

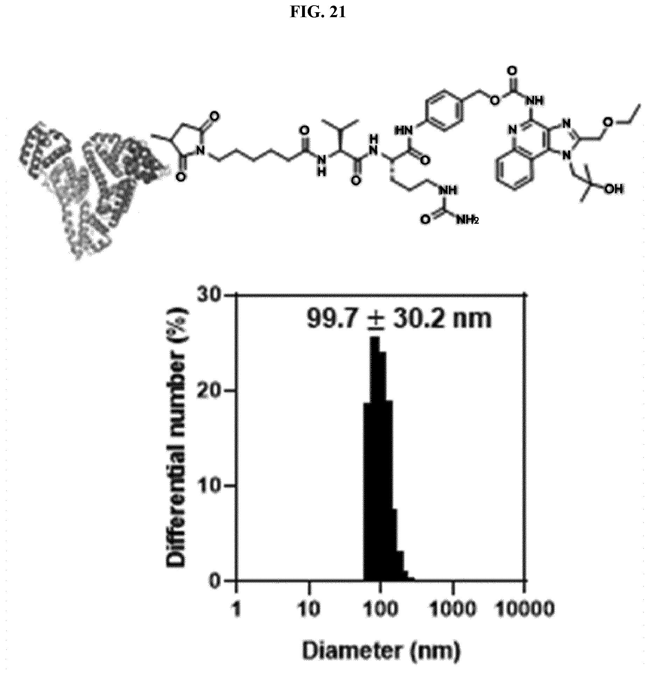

To synthesize a complex of a protein antigen, which is a type of sulfhydryl group-containing protein-based drug, and resiquimod, having a bond cleaved by cathepsin-B, first, resiquimod to which a linker binds to its active site was synthesized. In further detail, resiquimod (10 mg) and Mal-Val-Cit-PAB-PNP (47.2 mg) were added to 2 mL of N,N-dimethylformamide (DMF), and then dissolved. Afterward, N,N-diisopropylethylamine (DIPEA; 6.7 µL) and 1-hydroxybenzotriazole hydrate (1-HOBt; 315 µg) were added. Then, a mixture was prepared by stirring at room temperature for 16 hours. The OVA protein antigen (10 mg) was added to the prepared mixture and reacted, thereby preparing a complex, and then the resulting complex was purified by thin-layer chromatography, thereby preparing an OVA protein antigen-resiquimod complex having a bond cleaved by cathepsin-B. Then, the prepared complex was subjected to spectroscopy to confirm its size. The result is shown in FIG. 21.

As shown in FIG. 21, it was confirmed that the complex of the OVA protein antigen and resiquimod, having a bond cleaved by cathepsin-B, forms nanoparticles of approximately 99.7 nm in an aqueous solution.

From the above results, as shown in FIG. 22, the complex of the present invention may not exhibit toxicity because its activity is temporarily inhibited by connecting the functional drug to the toll-like receptor 7 or 8 agonist, and the secretion of an inflammatory cytokine and a non-specific immune response are inhibited when the complex is injected into the body, so side effects may be remarkably reduced, and then when the complex reached a desired location due to a physiological environment, the toll-like receptor 7 or 8 agonist and the functional drug may be separated, thereby increasing drug delivery capacity. It was also confirmed that, compared with when the toll-like receptor 7 or 8 agonist and the functional drug are used independently, and when the toll-like receptor 7 or 8 agonist and the functional drug are co-administered, the complex of the present invention can remarkably increase a therapeutic effect. Accordingly, the complex of the present invention in which the active site of the toll-like receptor 7 or 8 agonist and the functional drug are connected via a cleavable linker is expected to be effectively used in treatment of various diseases.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

Since a complex of the present invention, in which the active site of a toll-like receptor 7 or 8 agonist and a functional drug are connected via a cleavable linker, is temporarily inactive at time of administration into the body, but afterward, is activated by separating the toll-like receptor 7 or 8 agonist and the functional drug in a tumor microenvironment and/or under specific conditions in cells, it can reduce side effects such as a non-specific sensitive immune response, and since immune cells are effectively regulated by the toll-like receptor 7 or 8 agonist and the functional drug simultaneously acts, it can also have a remarkably increased therapeutic effect. Accordingly, it can be applied to all functional drugs capable of being co-administered with the toll-like receptor 7 or 8 agonist.

## Claims

1. A toll-like receptor 7/8 agonist-functional drug complex, in which the functional drug and the active site of the toll-like receptor 7 or 8 agonist are connected via a cleavable linker.

2. The complex of claim 1, wherein the functional drug acts first, and then the inactivated toll-like receptor 7 or 8 agonist acts when a linker is cleaved at an injection site, or in a tumor microenvironment or target cells and thus the active site is kinetically recovered.

3. The complex of claim 1, wherein the cleavable linker is cleaved at the chemical bond of a binding site due to any one or more factors selected from the group consisting of an enzyme, pH, redox potential, a temperature, ultrasonic wave, magnetic force, and a light source.

4. The complex of claim 1, wherein the cleavable linker is any one or more selected from the group consisting of a benzyl elimination-based linker, a trialkyl lockbased linker, a bicine-based linker, an acid labile linker, a lysosomally cleavable peptide and a cathepsin B-cleavable peptide.

5. The complex of claim 4, wherein the cleavable linker comprises any one or more bonds selected from the group consisting of carbamate, disulfide, hydrazine, ester, peptide, azide, β-glucuronide bonds, and a combination thereof.

6. The complex of claim 1, wherein the cleavable linker further comprises ethylene oxide or ethylene glycol at one or both ends thereof.

7. The complex of claim 1, wherein the toll-like receptor 7 or 8 agonist is any one or more selected from the group consisting of an imidazoquinoline-based agonist, an 8-hydroxyadenine-based agonist, a pteridone-based agonist, a 2-aminopyrimidinebased agonist, a benzoazepine-based agonist, and a 7-thia-8-oxoguanosine-based agonist.

8. The complex of claim 1, wherein the functional drug is any one or more selected from the group consisting of an antibody, an antibody fragment, a singlestranded antibody, an anticancer agent, an antigen, a cytokine, a protein, a peptide, an amino acid, an oligonucleotide, an enzyme, a lipid, a low-molecular compound, a glycoprotein, and a targeting ligand.

9. The complex of claim 1, wherein the functional drug is any one or more selected from the group consisting of an antibody, a protein-based drug, a sensitizer, and an anticancer agent.

10. A composition for controlling immune system, comprising the complex of claim 1 as an active ingredient.

11. The composition of claim 10, wherein the composition for controlling immune system activates one or more types of immune cells selected from the group consisting of antigen-presenting cells, B cells, natural killer (NK) cells, and T cells.

12. The composition of claim 10, wherein the composition for controlling immune system regulates the function of one or more types of immune cells selected from the group consisting of regulatory T (Treg) cells, myeloid derived suppressor cells (MDSCs), and M2 macrophages.

13. The composition of claim 10, further comprising one or more types of antibodies selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT, which target immune checkpoint factors.

14. A pharmaceutical composition for preventing or treating cancer, comprising the complex of claim 1 as an active ingredient.

15. The pharmaceutical composition of claim 14, further comprising:
a chemotherapeutic agent or immune checkpoint inhibitor.

16. The pharmaceutical composition of claim 14, the pharmaceutical composition inhibits cancer proliferation, metastasis, recurrence, or resistance to an anticancer therapy.

17. A method of preventing or treating cancer, comprising:
administering the composition comprising the complex of claim 1 as an active ingredient to a subject.

18. A composition for use in preventing or treating cancer, the composition comprising the complex of claim 1 as an active ingredient.

19. A use of the complex of claim 1 for the manufacture of medicament for preventing or treating cancer.
